# EUROPEAN PATENT APPLICATION

(11) **EP 3 415 526 A1**
(43) Date of publication of application: **19.12.2018**
(21) Application number: 18182275.0
(22) Date of filing: 10.12.2014
(51) Int. Cl.: C07K 14/605, A61K 38/26, C12Q 1/37, A61K 38/00, A61P 3/10

(54) **PROTEASE RESISTANT PEPTIDES**

(30) Priority: 13.12.2013 US 201361915662 P
(62) Divisional of application: 14809857.7
(71) Applicant: MedImmune Limited, Cambridge Cambridgeshire CB21 6GH (GB)
(72) Inventor: BEDNAREK, Maria, A, Cambridge, Cambridgeshire CB21 6GH (GB); REVELL, Jefferson, D, Cambridge, Cambridgeshire CB21 6GH (GB)
(74) Representative: AstraZeneca

(57) **Abstract**

The present invention provides protease-resistant peptides, methods of making such peptides, as well as compositions comprising protease-resistant peptides and method of treatment utilizing such peptides. Incorporation of alpha-methyl-functionalized amino acids directly into the main chain during standard peptide synthesis via the methodologies described herein has been determined to produce protease-resistant peptides. The preferred alpha-methyl-functionalized amino acid is alpha-methyl-Phenylalanine and the preferred peptide is glucagon-like peptide (GLP-1).

SEQUENCE LISTING

<110> MEDIMMUNE LIMITED

<120> PROTEASE-RESISTANT PEPTIDES

<130> ORPEP-100EP1

<140> PCT/EP2014/077240
<141> 2014-12-10

<150> 61/915,662
<151> 2013-12-13

<160> 63

<170> PatentIn version 3.5

<210> 1
<211> 30
<212> PRT
<213> Homo sapiens

<400> 1
His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg
            20                  25                  30

<210> 2
<211> 31
<212> PRT
<213> Artificial Sequence

<220>
<221> source
<223> /note="Description of Artificial Sequence: Synthetic
      polypeptide"

<220>
<221> MOD_RES
<222> (1)..(1)
<223> pGlu

<220>
<221> VARIANT
<222> (1)..(1)
<223> /replace=" "

<220>
<221> VARIANT
<222> (3)..(3)
<223> /replace="Aib" or "Pro" or "Gly"

<220>
<221> VARIANT
<222> (6)..(6)
<223> /replace="Pro" or "Ser"

<220>
<221> VARIANT
<222> (7)..(7)
<223> /replace="Aib" or "Bip" or "beta-beta-Dip" or "F5-Phe" or "PhG"
      or "Nle" or "homoPhe" or "homoTyr" or "N-Me-Phe" or "alpha-Me-
      Phe" or "alpha-Me-2F-Phe" or "Tyr" or "Trp" or "Tyr-OMe" or
      "4I-Phe" or "2F-Phe" or "3F-Phe" or "4F-Phe" or "1-Nal" or

<220>
<221> VARIANT
<222> (7)..(7)
<223> /replace="2-Nal" or "Pro" or "di-beta-beta-Me-Phe"

<220>
<221> VARIANT
<222> (14)..(14)
<223> /replace="Aib" or "Asp" or "Arg" or "Bip" or "Cha" or "beta-
      beta-Dip" or "Gln" or "F5-Phe" or "PhG" or "Nle" or "homoPhe"
      or "homoTyr" or "alpha-Me-Phe" or "alpha-Me-2F-Phe" or "Phe"
      or "Thr" or "Trp" or "Tyr-OMe" or "4I-Phe" or "2F-Phe" or

<220>
<221> VARIANT
<222> (14)..(14)
<223> /replace="3F-Phe" or "4FPhe" or "Tyr" or "1-Nal" or "2-Nal" or
      "Pro" or "di-beta-beta-Me-Phe" or "alpha-Me-Tyr" or "di-beta-
      beta-Me-Tyr"

<220>
<221> VARIANT
<222> (21)..(21)
<223> /replace="Aib" or "D-pro" or "Pro" or "alpha-Me-Lys" or
      "di-beta-beta-Me-Lys"

<220>
<221> VARIANT
<222> (23)..(23)
<223> /replace="Aib" or "Arg" or "Bip" or "Cha" or "Leu" or "Lys" or
      "2Cl-Phe" or "3Cl-Phe" or "4Cl-Phe" or "PhG" or "homoPhe" or
      "2Me-Phe" or "3Me-Phe" or "4Me-Phe" or "2CF3-Phe" or "3CF3-Phe"
      or "4CF3-Phe" or "beta-Phe" or "beta-Me-Phe" or "D-phe" or

<220>
<221> VARIANT
<222> (23)..(23)
<223> /replace="4I-Phe" or "3I-Phe" or "2F-Phe" or "beta-beta-Dip" or
      "beta-Ala" or "Nle" or "Leu" or "F5-Phe" or "homoTyr" or "alpha-
      Me-Phe" or "alpha-Me-2F-Phe" or "Ser" or "Tyr" or "Trp" or "Tyr-
      OMe" or "3F-Phe" or "4F-Phe" or "Pro" or "1-Nal" or "2-Nal" or

<220>
<221> VARIANT
<222> (23)..(23)
<223> /replace="di-beta-beta-Me-Phe" or "alpha-Me-Tyr" or "di-beta-
      beta-Me-Tyr" or "alpha-Me-Trp" or "di-beta-beta-Me-Trp"

<220>
<221> VARIANT
<222> (26)..(26)
<223> /replace="Aib" or "Bip" or "Cha" or "beta-beta-Dip" or "F5-Phe"
      or "PhG" or "Phe" or "Tyr" or "homoPhe" or "homoTyr" or "alpha-
      Me-Phe" or "alpha-Me-2F-Phe" or "2Me-Phe" or "3Me-Phe" or "4Me-
      Phe" or "Nle" or "Tyr-OMe" or "4I-Phe" or "1-Nal" or "2-Nal" or

<220>
<221> VARIANT
<222> (26)..(26)
<223> /replace="2F-Phe" or "3F-Phe" or "4F-Phe" or "Pro" or "N-Me-Trp"
      or "alpha-Me-Trp" or "di-beta-beta-Me-Trp" or "di-beta-beta-Me-
      Phe" or "alpha-Me-Tyr" or "di-beta-beta-Me-Tyr"

<220>
<221> VARIANT
<222> (27)..(27)
<223> /replace="Aib" or "Arg" or "Asp" or "Glu" or "Nle" or "Pro" or
      "Ser" or "N-Me-Leu" or "alpha-Me-Leu" or "Val" or "alpha-Me-Val"

<220>
<221> VARIANT
<222> (28)..(28)
<223> /replace="Aib" or "Lys" or "Pro" or "alpha-Me-Val" or
      "alpha-Me-Leu"

<220>
<221> VARIANT
<222> (29)..(29)
<223> /replace="Aib" or "Lys" or "Pro" or "alpha-Me-Lys"

<220>
<221> VARIANT
<222> (30)..(30)
<223> /replace="Aib" or "Pro" or "Ser"

<220>
<221> VARIANT
<222> (31)..(31)
<223> /replace="Aib" or "Glu" or "Lys" or "Pro" or "alpha-Me-Arg"
      or "alpha-Me-Lys"

<220>
<221> misc_feature
<222> (1)..(31)
<223> /note="Variant residues given in the sequence have no
      preference with respect to those in the annotations
      for variant positions"

<400> 2
Glu His Ala Glu Gly Thr Phe Thr Ser Asp Val Ser Ser Ala Leu Glu
1               5                   10                  15

Gly Gln Ala Ala Lys Glu Asp Ile Ala Arg Ala Glu Glu Glu Gly
            20                  25                  30

<210> 3
<211> 30
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<400> 3
His Xaa Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg
            20                  25                  30

<210> 4
<211> 30
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<400> 4
His Xaa Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg
            20                  25                  30

<210> 5
<211> 31
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<400> 5
His Xaa Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Lys
            20                  25                  30

<210> 6
<211> 30
<212> PRT
<213> Artificial Sequence

<220>
<221> source
<223> /note="Description of Artificial Sequence: Synthetic
      polypeptide"


<220>
<221> MOD_RES
<222> (2)..(2)
<223> Aib

<400> 6
His Xaa Glu Gly Thr Phe Thr Ser Asp Val Ser Ser Tyr Leu Glu Gly
1               5                   10                  15


Gln Ala Ala Lys Glu Phe Ile Ala Trp Leu Val Lys Gly Arg
            20                  25                  30


<210> 7
<211> 30
<212> PRT
<213> Artificial Sequence

<220>
<221> source
<223> /note="Description of Artificial Sequence: Synthetic
      polypeptide"


<220>
<221> MOD_RES
<222> (2)..(2)
<223> Aib

<220>
<221> MOD_RES
<222> (6)..(6)
<223> Aib

<400> 7
His Xaa Glu Gly Thr Xaa Thr Ser Asp Val Ser Ser Tyr Leu Glu Gly
1               5                   10                  15


Gln Ala Ala Lys Glu Phe Ile Ala Trp Leu Val Lys Gly Arg
            20                  25                  30


<210> 8
<211> 30
<212> PRT
<213> Artificial Sequence

<220>
<221> source

<223> /note="Description of Artificial Sequence: Synthetic
      polypeptide"

<220>
<221> MOD_RES
<222> (2)..(2)
<223> Aib

<400> 8
His Xaa Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg
            20                  25                  30

<210> 9
<211> 30
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<400> 9
His Xaa Glu Gly Thr Phe Thr Ser Asp Val Ser Ser Phe Leu Glu Gly
1               5                   10                  15

Gln Ala Ala Lys Glu Phe Ile Ala Trp Leu Val Lys Gly Arg
            20                  25                  30

<210> 10
<211> 30
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<220>
<221> MOD_RES
<222> (13)..(13)
<223> Aib

<400> 10
His Xaa Glu Gly Thr Phe Thr Ser Asp Val Ser Ser Xaa Leu Glu Gly
1               5                   10                  15

Gln Ala Ala Lys Glu Phe Ile Ala Trp Leu Val Lys Gly Arg
            20                  25                  30

<210> 11
<211> 30
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<400> 11
His Xaa Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg
            20                  25                  30

<210> 12
<211> 30
<212> PRT
<213> Artificial Sequence

<220>
<221> source
<223> /note="Description of Artificial Sequence: Synthetic
      peptide"

<220>
<221> MOD_RES
<222> (2)..(2)
<223> Aib

<220>
<221> MOD_RES
<222> (22)..(22)
<223> Aib

<400> 12
His Xaa Glu Gly Thr Phe Thr Ser Asp Val Ser Ser Tyr Leu Glu Gly
1               5                   10                  15

Gln Ala Ala Lys Glu Xaa Ile Ala Trp Leu Val Lys Gly Arg
            20                  25                  30

<210> 13
<211> 30
<212> PRT
<213> Artificial Sequence

<220>
<221> source
<223> /note="Description of Artificial Sequence: Synthetic
      polypeptide"


<220>
<221> MOD_RES
<222> (2)..(2)
<223> Aib

<400> 13
His Xaa Glu Gly Thr Phe Thr Ser Asp Val Ser Ser Tyr Leu Glu Gly
1               5                   10                  15


Gln Ala Ala Lys Glu Phe Ile Ala Trp Leu Val Lys Gly Arg
            20                  25                  30


<210> 14
<211> 30
<212> PRT
<213> Artificial Sequence

<220>
<221> source
<223> /note="Description of Artificial Sequence: Synthetic
      polypeptide"


<220>
<221> MOD_RES
<222> (2)..(2)
<223> Aib

<400> 14
His Xaa Glu Gly Thr Phe Thr Ser Asp Val Ser Ser Tyr Leu Glu Gly
1               5                   10                  15


Gln Ala Ala Lys Glu Phe Ile Ala Phe Leu Val Lys Gly Arg
            20                  25                  30


<210> 15
<211> 30
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<220>
<221> MOD_RES
<222> (25)..(25)
<223> Aib

<400> 15
His Xaa Glu Gly Thr Phe Thr Ser Asp Val Ser Ser Tyr Leu Glu Gly
1               5                   10                  15

Gln Ala Ala Lys Glu Phe Ile Ala Xaa Leu Val Lys Gly Arg
            20                  25                  30

<210> 16
<211> 30
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<220>
<221> MOD_RES
<222> (6)..(6)
<223> Aib

<220>
<221> MOD_RES
<222> (13)..(13)
<223> Aib

<220>
<221> MOD_RES
<222> (22)..(22)
<223> Aib

<220>
<221> MOD_RES
<222> (25)..(25)
<223> Aib

<400> 16
His Xaa Glu Gly Thr Xaa Thr Ser Asp Val Ser Ser Xaa Leu Glu Gly
1               5                   10                  15

Gln Ala Ala Lys Glu Xaa Ile Ala Xaa Leu Val Lys Gly Arg
            20                  25                  30

<210> 17
<211> 30
<212> PRT
<213> Artificial Sequence

<220>
<221> source
<223> /note="Description of Artificial Sequence: Synthetic
      polypeptide"


<220>
<221> MOD_RES
<222> (2)..(2)
<223> Aib

<220>
<221> MOD_RES
<222> (6)..(6)
<223> Nle

<220>
<221> MOD_RES
<222> (13)..(13)
<223> Nle

<220>
<221> MOD_RES
<222> (22)..(22)
<223> Nle

<220>
<221> MOD_RES
<222> (25)..(25)
<223> Nle

<400> 17
His Xaa Glu Gly Thr Xaa Thr Ser Asp Val Ser Ser Xaa Leu Glu Gly
1               5                   10                  15


Gln Ala Ala Lys Glu Xaa Ile Ala Xaa Leu Val Lys Gly Arg
            20                  25                  30


<210> 18
<211> 30
<212> PRT
<213> Artificial Sequence

<220>
<221> source
<223> /note="Description of Artificial Sequence: Synthetic
      polypeptide"


<220>
<221> MOD_RES
<222> (2)..(2)
<223> Aib

<400> 18

His Xaa Glu Gly Thr Phe Thr Ser Asp Val Ser Ser Phe Leu Glu Gly
1               5                   10                  15

Gln Ala Ala Lys Glu Phe Ile Ala Phe Leu Val Lys Gly Arg
            20                  25                  30

<210> 19
<211> 30
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<400> 19
His Xaa Glu Gly Thr Phe Thr Ser Asp Val Ser Ser Phe Leu Glu Gly
1               5                   10                  15

Gln Ala Ala Lys Glu Phe Ile Ala Phe Leu Val Lys Gly Arg
            20                  25                  30

<210> 20
<211> 30
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<400> 20
His Xaa Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg
            20                  25                  30

<210> 21
<211> 30
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<220>
<221> MOD_RES
<222> (20)..(20)
<223> Aib

<400> 21
His Xaa Glu Gly Thr Phe Thr Ser Asp Val Ser Ser Tyr Leu Glu Gly
1               5                   10                  15

Gln Ala Ala Xaa Glu Phe Ile Ala Trp Leu Val Lys Gly Arg
            20                  25                  30

<210> 22
<211> 30
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<400> 22
His Xaa Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg
            20                  25                  30

<210> 23
<211> 30
<212> PRT
<213> Artificial Sequence

<220>
<221> source
<223> /note="Description of Artificial Sequence: Synthetic
      polypeptide"

<220>
<221> MOD_RES

<222> (2)..(2)
<223> Aib

<220>
<221> MOD_RES
<222> (28)..(28)
<223> Aib

<400> 23
His Xaa Glu Gly Thr Phe Thr Ser Asp Val Ser Ser Tyr Leu Glu Gly
1               5                   10                  15

Gln Ala Ala Lys Glu Phe Ile Ala Trp Leu Val Xaa Gly Arg
            20                  25                  30

<210> 24
<211> 30
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<400> 24
His Xaa Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg
            20                  25                  30

<210> 25
<211> 30
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<220>
<221> MOD_RES
<222> (30)..(30)
<223> Aib

<400> 25
His Xaa Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Xaa
            20                  25                  30

<210> 26
<211> 30
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<400> 26
His Xaa Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg
            20                  25                  30

<210> 27
<211> 30
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<220>
<221> MOD_RES
<222> (20)..(20)
<223> Aib

<220>
<221> MOD_RES
<222> (28)..(28)
<223> Aib

<220>
<221> MOD_RES
<222> (30)..(30)

<223> Aib

<400> 27
His Xaa Glu Gly Thr Phe Thr Ser Asp Val Ser Ser Tyr Leu Glu Gly
1               5                   10                  15

Gln Ala Ala Xaa Glu Phe Ile Ala Trp Leu Val Xaa Gly Xaa
            20                  25                  30

<210> 28
<211> 30
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<400> 28
His Xaa Glu Gly Ser Phe Thr Ser Asp Val Ser Ser Tyr Leu Glu Gly
1               5                   10                  15

Gln Ala Ala Lys Glu Phe Ile Ala Trp Leu Val Lys Gly Arg
            20                  25                  30

<210> 29
<211> 30
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<400> 29
His Xaa Glu Gly Thr Phe Thr Ser Asp Val Ser Ser Phe Leu Glu Gly
1               5                   10                  15

Gln Ala Ala Lys Glu Phe Ile Ala Trp Leu Val Lys Gly Arg
            20                  25                  30

<210> 30
<211> 30

<212> PRT
<213> Artificial Sequence

<220>
<221> source
<223> /note="Description of Artificial Sequence: Synthetic
      polypeptide"


<220>
<221> MOD_RES
<222> (2)..(2)
<223> Aib

<400> 30
His Xaa Glu Gly Thr Phe Thr Ser Asp Val Ser Ser Phe Leu Glu Gly
1               5                   10                  15


Gln Ala Ala Lys Glu Phe Ile Ala Phe Leu Val Lys Gly Arg
            20                  25                  30


<210> 31
<211> 30
<212> PRT
<213> Artificial Sequence

<220>
<221> source
<223> /note="Description of Artificial Sequence: Synthetic
      polypeptide"


<220>
<221> MOD_RES
<222> (2)..(2)
<223> Aib

<400> 31
His Xaa Glu Gly Thr Phe Thr Ser Asp Val Ser Ser Tyr Leu Glu Gly
1               5                   10                  15


Gln Ala Ala Lys Glu Phe Ile Ala Phe Leu Val Lys Gly Arg
            20                  25                  30


<210> 32
<211> 30
<212> PRT
<213> Artificial Sequence

<220>
<221> source
<223> /note="Description of Artificial Sequence: Synthetic
      polypeptide"


<220>
<221> MOD_RES
<222> (2)..(2)
<223> Aib

<400> 32
His Xaa Glu Gly Thr Phe Thr Ser Asp Val Ser Ser Phe Leu Glu Gly
1               5                   10                  15

Gln Ala Ala Lys Glu Phe Ile Ala Phe Leu Val Lys Gly Arg
            20                  25                  30

<210> 33
<211> 30
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<400> 33
His Xaa Glu Gly Ser Phe Thr Ser Asp Val Ser Ser Phe Leu Glu Gly
1               5                   10                  15

Gln Ala Ala Lys Glu Phe Ile Ala Phe Leu Val Lys Gly Arg
            20                  25                  30

<210> 34
<211> 30
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<400> 34
His Xaa Glu Gly Ser Phe Thr Ser Asp Val Ser Ser Phe Leu Glu Gly
1               5                   10                  15

Gln Ala Ala Lys Glu Phe Ile Ala Trp Leu Val Lys Gly Arg
            20                  25                  30

<210> 35
<211> 30
<212> PRT

<213> Artificial Sequence

<220>
<221> source
<223> /note="Description of Artificial Sequence: Synthetic
      polypeptide"

<220>
<221> MOD_RES
<222> (2)..(2)
<223> Aib

<400> 35
His Xaa Glu Gly Ser Phe Thr Ser Asp Val Ser Ser Phe Leu Glu Gly
1               5                   10                  15

Gln Ala Ala Lys Glu Phe Ile Ala Phe Leu Val Lys Gly Arg
            20                  25                  30

<210> 36
<211> 30
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<400> 36
His Xaa Glu Gly Ser Phe Thr Ser Asp Val Ser Ser Tyr Leu Glu Gly
1               5                   10                  15

Gln Ala Ala Lys Glu Phe Ile Ala Phe Leu Val Lys Gly Arg
            20                  25                  30

<210> 37
<211> 30
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<400> 37
His Xaa Glu Gly Ser Phe Thr Ser Asp Val Ser Ser Phe Leu Glu Gly
1               5                   10                  15

Gln Ala Ala Lys Glu Phe Ile Ala Phe Leu Val Lys Gly Arg
            20                  25                  30

<210> 38
<211> 30
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<400> 38
His Xaa Glu Gly Ser Phe Thr Ser Asp Val Ser Ser Phe Leu Glu Gly
1               5                   10                  15

Gln Ala Ala Lys Glu Phe Ile Ala Phe Leu Val Lys Gly Arg
            20                  25                  30

<210> 39
<211> 30
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<400> 39
His Xaa Glu Gly Ser Phe Thr Ser Asp Val Ser Ser Phe Leu Glu Gly
1               5                   10                  15

Gln Ala Ala Lys Glu Phe Ile Ala Phe Leu Val Lys Gly Gly
            20                  25                  30

<210> 40
<211> 30
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<220>
<221> MOD_RES
<222> (27)..(27)
<223> Aib

<400> 40
His Xaa Glu Gly Ser Phe Thr Ser Asp Val Ser Ser Phe Leu Glu Gly
1               5                   10                  15

Gln Ala Ala Lys Glu Phe Ile Ala Phe Leu Xaa Lys Gly Arg
            20                  25                  30

<210> 41
<211> 30
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<400> 41
His Xaa Glu Gly Ser Phe Thr Ser Asp Val Ser Ser Phe Leu Glu Gly
1               5                   10                  15

Gln Ala Ala Lys Glu Phe Ile Ala Phe Leu Val Lys Gly Arg
            20                  25                  30

<210> 42
<211> 30
<212> PRT
<213> Artificial Sequence

<220>
<221> source
<223> /note="Description of Artificial Sequence: Synthetic
      polypeptide"

<220>

<221> MOD_RES
<222> (2)..(2)
<223> Aib

<220>
<221> MOD_RES
<222> (29)..(29)
<223> Aib

<400> 42
His Xaa Glu Gly Ser Phe Thr Ser Asp Val Ser Ser Phe Leu Glu Gly
1               5                   10                  15

Gln Ala Ala Lys Glu Phe Ile Ala Phe Leu Val Lys Xaa Arg
            20                  25                  30

<210> 43
<211> 30
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<220>
<221> MOD_RES
<222> (27)..(27)
<223> Aib

<220>
<221> MOD_RES
<222> (29)..(29)
<223> Aib

<400> 43
His Xaa Glu Gly Ser Phe Thr Ser Asp Val Ser Ser Phe Leu Glu Gly
1               5                   10                  15

Gln Ala Ala Lys Glu Phe Ile Ala Phe Leu Xaa Lys Xaa Arg
            20                  25                  30

<210> 44
<211> 30
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<400> 44
His Xaa Glu Gly Ser Phe Thr Ser Asp Val Ser Ser Phe Leu Glu Gly
1               5                   10                  15

Gln Ala Ala Lys Glu Phe Ile Ala Phe Leu Val Lys Gly Gly
            20                  25                  30

<210> 45
<211> 30
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<400> 45
His Xaa Glu Gly Ser Phe Thr Ser Asp Val Ser Ser Phe Leu Glu Gly
1               5                   10                  15

Gln Ala Ala Lys Glu Phe Ile Ala Phe Leu Val Lys Gly Gly
            20                  25                  30

<210> 46
<211> 30
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<400> 46
His Xaa Glu Gly Ser Phe Thr Ser Asp Val Ser Ser Phe Leu Glu Gly
1               5                   10                  15

Gln Ala Ala Lys Glu Phe Ile Ala Phe Leu Val Lys Gly Gly
            20                  25                  30

<210> 47
<211> 31
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<400> 47
His Xaa Glu Gly Ser Phe Thr Ser Asp Val Ser Ser Phe Leu Glu Gly
1               5                   10                  15

Gln Ala Ala Lys Glu Phe Ile Ala Phe Leu Val Lys Gly Gly Lys
            20                  25                  30

<210> 48
<211> 40
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<400> 48
His Xaa Glu Gly Ser Phe Thr Ser Asp Val Ser Ser Phe Leu Glu Gly
1               5                   10                  15

Gln Ala Ala Lys Glu Phe Ile Ala Phe Leu Val Lys Gly Gly Ser Ser
            20                  25                  30

Gly Ser Ser Gly Ser Ser Gly Lys
        35                  40

<210> 49
<211> 40
<212> PRT
<213> Artificial Sequence

<220>
<221> source

<223> /note="Description of Artificial Sequence: Synthetic
      polypeptide"

<220>
<221> MOD_RES
<222> (2)..(2)
<223> Aib

<400> 49
His Xaa Glu Gly Ser Phe Thr Ser Asp Val Ser Ser Phe Leu Glu Gly
1               5                   10                  15

Gln Ala Ala Lys Glu Phe Ile Ala Phe Leu Val Lys Gly Gly Ser Ser
            20                  25                  30

Gly Ser Ser Gly Ser Ser Gly Lys
        35                  40

<210> 50
<211> 40
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<400> 50
His Xaa Glu Gly Ser Phe Thr Ser Asp Val Ser Ser Phe Leu Glu Gly
1               5                   10                  15

Gln Ala Ala Lys Glu Phe Ile Ala Phe Leu Val Lys Gly Gly Ser Ser
            20                  25                  30

Gly Ser Ser Gly Ser Ser Gly Cys
        35                  40

<210> 51
<211> 30
<212> PRT
<213> Artificial Sequence

<220>
<221> source
<223> /note="Description of Artificial Sequence: Synthetic
      polypeptide"

<220>

<221> MOD_RES
<222> (2)..(2)
<223> Aib

<400> 51
His Xaa Glu Gly Ser Phe Thr Ser Asp Val Ser Ser Phe Leu Glu Gly
1               5                   10                  15

Gln Ala Ala Lys Glu Phe Ile Ala Phe Val Val Lys Gly Gly
            20                  25                  30

<210> 52
<211> 31
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<400> 52
His Xaa Glu Gly Ser Phe Thr Ser Asp Val Ser Ser Phe Leu Glu Gly
1               5                   10                  15

Gln Ala Ala Lys Glu Phe Ile Ala Phe Val Val Lys Gly Gly Lys
            20                  25                  30

<210> 53
<211> 31
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<400> 53
His Xaa Glu Gly Ser Phe Thr Ser Asp Val Ser Ser Phe Leu Glu Gly
1               5                   10                  15

Gln Ala Ala Lys Glu Phe Ile Ala Phe Val Val Lys Gly Gly Lys
            20                  25                  30

<210> 54
<211> 31
<212> PRT
<213> Artificial Sequence

<220>
<221> source
<223> /note="Description of Artificial Sequence: Synthetic
      polypeptide"


<220>
<221> MOD_RES
<222> (2)..(2)
<223> Aib

<400> 54
His Xaa Glu Gly Ser Phe Thr Ser Asp Val Ser Ser Phe Leu Glu Gly
1               5                   10                  15


Gln Ala Ala Lys Glu Phe Ile Ala Phe Val Val Lys Gly Gly Lys
            20                  25                  30


<210> 55
<211> 31
<212> PRT
<213> Artificial Sequence

<220>
<221> source
<223> /note="Description of Artificial Sequence: Synthetic
      polypeptide"


<220>
<221> MOD_RES
<222> (2)..(2)
<223> Aib

<400> 55
His Xaa Glu Gly Ser Phe Thr Ser Asp Val Ser Ser Phe Leu Glu Gly
1               5                   10                  15


Gln Ala Ala Lys Glu Phe Ile Ala Phe Val Val Lys Gly Gly Lys
            20                  25                  30


<210> 56
<211> 31
<212> PRT
<213> Artificial Sequence

<220>
<221> source
<223> /note="Description of Artificial Sequence: Synthetic
      polypeptide"


<220>
<221> MOD_RES

<222> (2)..(2)
<223> Aib

<400> 56
His Xaa Glu Gly Ser Phe Thr Ser Asp Val Ser Ser Phe Leu Glu Gly
1               5                   10                  15

Gln Ala Ala Lys Glu Phe Ile Ala Phe Val Val Lys Gly Gly Lys
            20                  25                  30

<210> 57
<211> 30
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<400> 57
His Xaa Glu Gly Ser Phe Thr Ser Asp Val Ser Ser Tyr Leu Glu Gly
1               5                   10                  15

Gln Ala Ala Lys Glu Phe Ile Ala Trp Leu Val Lys Gly Arg
            20                  25                  30

<210> 58
<211> 31
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<400> 58
His Xaa Glu Gly Ser Phe Thr Ser Asp Val Ser Ser Tyr Leu Glu Gly
1               5                   10                  15

Gln Ala Ala Lys Glu Phe Ile Ala Trp Leu Val Lys Gly Arg Lys
            20                  25                  30

<210> 59

<211> 31
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<400> 59
His Xaa Glu Gly Ser Phe Thr Ser Asp Val Ser Ser Phe Leu Glu Gly
1               5                   10                  15

Glu Ala Ala Lys Glu Phe Ile Ala Phe Val Val Lys Gly Gly Lys
            20                  25                  30

<210> 60
<211> 30
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<400> 60
His Xaa Glu Gly Ser Phe Thr Ser Asp Val Ser Ser Phe Leu Glu Gly
1               5                   10                  15

Glu Ala Ala Lys Glu Phe Ile Ala Phe Val Val Lys Gly Gly
            20                  25                  30

<210> 61
<211> 31
<212> PRT
<213> Artificial Sequence

<220>
<221> source
<223> /note="Description of Artificial Sequence: Synthetic
      polypeptide"

<220>
<221> MOD_RES
<222> (2)..(2)

<223> Aib

<400> 61
His Xaa Glu Gly Ser Phe Thr Ser Asp Val Ser Ser Phe Leu Glu Gly
1               5                   10                  15

Glu Ala Ala Lys Glu Phe Ile Ala Phe Val Val Lys Gly Gly Lys
            20                  25                  30

<210> 62
<211> 30
<212> PRT
<213> Artificial Sequence

<220>
<221> source
<223> /note="Description of Artificial Sequence: Synthetic
      polypeptide"

<220>
<221> MOD_RES
<222> (2)..(2)
<223> Aib

<400> 62
His Xaa Glu Gly Thr Phe Thr Ser Asp Val Ser Ser Phe Leu Glu Gly
1               5                   10                  15

Gln Ala Ala Lys Glu Phe Ile Ala Phe Leu Val Lys Gly Arg
            20                  25                  30

<210> 63
<211> 31
<212> PRT
<213> Artificial Sequence

<220>
<221> source
<223> /note="Description of Artificial Sequence: Synthetic
      polypeptide"

<220>
<221> MOD_RES
<222> (1)..(1)
<223> pGlu

<220>
<221> VARIANT
<222> (1)..(1)
<223> /replace=" "

<220>
<221> VARIANT
<222> (3)..(3)
<223> /replace="Aib" or "Pro" or "Gly"

<220>

<221> VARIANT
<222> (6)..(6)
<223> /replace="Pro" or "Ser"

<220>
<221> VARIANT
<222> (7)..(7)
<223> /replace="Aib" or "Bip" or "beta-beta-Dip" or "F5-Phe" or
      "PhG" or "Nle" or "homoPhe" or "homoTyr" or "N-Me-Phe" or
      "alpha-Me-Phe" or "alpha-Me-2F-Phe" or "Tyr" or "Trp" or
      "Tyr-OMe" or "4I-Phe" or "2F-Phe" or "3F-Phe" or

<220>
<221> VARIANT
<222> (7)..(7)
<223> /replace="4F-Phe" or "1-Nal" or "2-Nal" or "Pro" or
      "di-beta-beta-Me-Phe"

<220>
<221> VARIANT
<222> (14)..(14)
<223> /replace="Aib" or "Asp" or "Arg" or "Bip" or "Cha" or "beta-
      beta-Dip" or "Gln" or "F5-Phe" or "PhG" or "Nle" or "homoPhe"
      or "homoTyr" or "alpha-Me-Phe" or "alpha-Me-2F-Phe" or "Phe"
      or "Thr" or "Trp" or "Tyr-OMe" or "4I-Phe" or "2F-Phe" or

<220>
<221> VARIANT
<222> (14)..(14)
<223> /replace="3F-Phe" or "4FPhe" or "Tyr" or "1-Nal" or "2-Nal"
      or "Pro" or "di-beta-beta-Me-Phe"

<220>
<221> VARIANT
<222> (21)..(21)
<223> /replace="Aib" or "D-pro" or "Pro" or "alpha-Me-Lys"

<220>
<221> VARIANT
<222> (23)..(23)
<223> /replace="Aib" or "Arg" or "Bip" or "Cha" or "Leu" or "Lys" or
      "2Cl-Phe" or "3Cl-Phe" or "4Cl-Phe" or "PhG" or "homoPhe" or
      "2Me-Phe" or "3Me-Phe" or "4Me-Phe" or "2CF3-Phe" or "3CF3-Phe"
      or "4CF3-Phe" or "beta-Phe" or "beta-Me-Phe" or "D-phe" or

<220>
<221> VARIANT
<222> (23)..(23)
<223> /replace="4I-Phe" or "3I-Phe" or "2F-Phe" or "beta-beta-Dip"
      or "beta-Ala" or "Nle" or "Leu" or "F5-Phe" or "homoTyr" or
      "alpha-Me-Phe" or "alpha-Me-2F-Phe" or "Ser" or "Tyr" or
      "Trp" or "Tyr-OMe" or "3F-Phe" or "4F-Phe" or "Pro" or

<220>
<221> VARIANT
<222> (23)..(23)
<223> /replace="1-Nal" or "2-Nal" or "di-beta-beta-Me-Phe"

<220>
<221> VARIANT
<222> (26)..(26)
<223> /replace="Aib" or "Bip" or "Cha" or "beta-beta-Dip" or "F5-Phe"
      or "PhG" or "Phe" or "Tyr" or "homoPhe" or "homoTyr" or "alpha-

Me-Phe" or "alpha-Me-2F-Phe" or "2Me-Phe" or "3Me-Phe" or "4Me-
Phe" or "Nle" or "Tyr-OMe" or "4I-Phe" or "1-Nal" or "2-Nal" or

<220>
<221> VARIANT
<222> (26)..(26)
<223> /replace="2F-Phe" or "3F-Phe" or "4F-Phe" or "Pro" or "N-Me-Trp"
or "alpha-Me-Trp" or "di-beta-beta-Me-Phe"

<220>
<221> VARIANT
<222> (27)..(27)
<223> /replace="Aib" or "Arg" or "Asp" or "Glu" or "Nle" or "Pro"
or "Ser" or "N-Me-Leu" or "alpha-Me-Leu" or "Val"

<220>
<221> VARIANT
<222> (28)..(28)
<223> /replace="Aib" or "Lys" or "alpha-Me-Val" or "Pro"

<220>
<221> VARIANT
<222> (29)..(29)
<223> /replace="Aib" or "alpha-Me-Lys" or "Pro"

<220>
<221> VARIANT
<222> (30)..(30)
<223> /replace="Aib" or "Pro" or "Ser"

<220>
<221> VARIANT
<222> (31)..(31)
<223> /replace="Aib" or "Glu" or "Pro" or "alpha-Me-Arg"

<220>
<221> misc_feature
<222> (1)..(31)
<223> /note="Variant residues given in the sequence have no
preference with respect to those in the annotations
for variant positions"

<400> 63
Glu His Ala Glu Gly Thr Phe Thr Ser Asp Val Ser Ser Ala Leu Glu
1               5                   10                  15

Gly Gln Ala Ala Lys Glu Asp Ile Ala Arg Ala Glu Glu Glu Gly
            20                  25                  30

## Description

### BACKGROUND OF THE INVENTION

### FIELD OF THE INVENTION

The present invention provides protease-resistant peptides, methods of making such peptides, as well as compositions comprising protease-resistant peptides and methods of treatment utilizing such peptides. Incorporation of alpha-methyl-functionalized amino acids directly into the main chain during standard peptide synthesis via the methodologies described herein.

### BACKGROUND ART

The development of long-acting peptide therapeutics is hampered by factors such as short plasma half-life and poor oral bioavailability, largely a result of the natural susceptibility of peptides to enzymatic degradation. The majority of proteolytic functions are necessary, including regulating essential biomolecular processes such as turning off peptide signaling events at cell surfaces, or the gastric breakdown of proteins and peptides during digestion. Thus, the activity of the responsible proteases cannot simply be inhibited without, in many cases, causing other metabolic disturbances.

In order to overcome degradation, increasing the enzymatic resistance of a peptide of interest is therefore desirable. Generally, two primary methods are utilized to increase enzymatic resistance: sequence specific modifications, i.e. those affecting the primary structure of the peptide itself; and globally effective modifications, i.e. those which alter certain overall physicochemical characteristics of the peptide. Introduced strategically, such modifications may reduce the effects of natural physiological processes which would otherwise eliminate or inactivate a peptide whose action is desired, e.g. enzymatic degradation and/or clearance by renal ultrafiltration.

Sequence specific modifications include incorporation of proteolysis-resistant unusual amino acids, or more involved modifications including cyclization between naturally occurring side-chain functions, e.g. disulfide formation (Cys-Cys), or lactamization (Lys-Glu or Lys-Asp). Additional modifications include cyclization between unnatural amino acid surrogates within the peptide backbone e.g. olefin metathesis stapling.

Global modifications include processes such as peptide lipidation e.g. palmitoylation and/or PEGylation. Palmitoylation has the effect of creating a circulating reservoir of peptide which weakly associates with naturally abundant albumin in blood serum. Peptide associated with albumin effectively escapes renal ultrafiltration since the size of the associated complex is above the glomerular filtration cutoff. As the peptide dissociates from the surface of the albumin it is again free to interact with endogenous receptors. PEGylation has the effect of physically shielding the peptide from proteolysis and imparts significant hydrophilicity which upon hydration greatly increases the hydrodynamic radius of the therapeutic molecule to overcome renal clearance. However, neither lipidation nor PEGylation have a significant impact on the susceptibility of the main peptide chain towards proteolysis.

While these technologies may be broadly applicable to therapeutic peptides in general, and to an extent are able to extend circulatory half-life, a need still exists for methods of increasing stability of peptides and proteins to enzymatic degradation, particularly in light of the desire to produce orally administrable peptides.

### BRIEF SUMMARY OF THE INVENTION

Described throughout are embodiments that meet the needs described above.

In one embodiment synthetic peptides are provided comprising at least one substitution of an alpha-methyl functionalized amino acid for a native amino acid residue. Suitably, the synthetic peptide maintains substantially the same receptor potency and selectivity as a corresponding synthetic peptide that does not comprise any substitutions.

In embodiments, the at least one alpha-methyl functionalized amino acid corresponds to the substituted native amino acid residue. Suitably, the at least one alpha-methyl functionalized amino acid includes alpha-methyl Histidine, alpha-methyl Alanine, alpha-methyl Isoleucine, alpha-methyl Arginine, alpha-methyl Leucine, alpha-methyl Asparagine, alpha-methyl Lysine, alpha-methyl Aspartic acid, alpha-methyl Methionine, alpha-methyl Cysteine, alpha-methyl Phenylalanine, alpha-methyl Glutamic acid, alpha-methyl Threonine, alpha-methyl Glutamine, alpha-methyl Tryptophan, alpha-methyl Glycine, alpha-methyl Valine, alpha-methyl Ornithine, alpha-methyl Proline, alpha-methyl Selenocysteine, alpha-methyl Serine and/or alpha-methyl Tyrosine.

In embodiments, the synthetic peptide is substantially resistant to proteolytic degradation, including for example, DPP-IV, neprilysin, chymotrypsin, plasmin, thrombin, kallikrein, elastase, trypsin and/or pepsin degradation.

Suitably, the native amino acid residue is a site susceptible to proteolytic cleavage.

In embodiments, the peptide is an incretin class peptide, including but not limited to, a glucagon-like peptide 1 (GLP-1), a glucose-dependent insulinotropic peptide (GIP), an exenatide peptide plus glucagon, secretins, tenomodulin, oxyntomodulin and vasoactive intestinal peptide (VIP).

In other embodiments, the peptide is insulin.

In further embodiments, a GLP-1 peptide is provided, suitably comprising at least three substitutions of alpha-methyl functionalized amino acids for native amino acid residues, wherein the synthetic GLP-1 peptide maintains substantially the same receptor potency and selectivity as a corresponding synthetic GLP-1 peptide that does not comprise the substitutions. In embodiments, at least three, or at least four, alpha-methyl functionalized amino acids are alpha-methyl phenylalanine. Suitably, the alpha-methyl functionalized amino acids are alpha-methyl phenylalanine substituted at positions Phe6, Try13, Phe22 and Trp25.

Suitably, the GLP-1 peptides further comprise an aminoisobutyric acid substitution at position 2 (Aib2), a serine modification at position 5 (Ser5), an alpha-methyl lysine substituted at positions 20 (α-MeLys20) and 28 (α-MeLys28), a valine modification position 26 (Val26), and/or a carboxy-terminal lipidation or PEGylation.

Also provided are methods of preparing a synthetic peptide, comprising identifying at least one native amino acid residue in the peptide for substitution, and substituting an alpha-methyl functionalized amino acid for the identified native amino acid residue. Suitably, the synthetic peptide maintains substantially the same receptor potency and selectivity as a corresponding synthetic peptide that does not comprise the substitution, and wherein the synthetic peptide is substantially resistant to proteolytic degradation.

In further embodiments, methods of preparing a proteolytically stable peptide are provided, comprising exposing a peptide to one or more proteases, identifying at least one native amino acid residue which is a site susceptible to proteolytic cleavage, and substituting an alpha-methyl functionalized amino acid for the identified amino acid residue. Suitably, the synthetic peptide maintains substantially the same receptor potency and selectivity as a corresponding synthetic peptide that does not comprise the substitution, and wherein the synthetic peptide is substantially resistant to proteolytic degradation.

Also provided are methods of treating a patient, comprising administering a pharmaceutically effective amount of a synthetic peptide as described herein.

In further embodiments, a synthetic GLP-1 peptide comprising the following amino acid sequence is provided:

R¹-His-X1-Glu-Gly-X2-X3-Thr-Ser-Asp-Val-Ser-Ser-X4-Leu-Glu-Gly-Gln-Ala-Ala-X5-Glu-X6-Ile-Ala-X7-X8-X9-X10-X11-X12-R² (SEQ ID NO:2),

wherein:
R¹ is Hy, Ac or *p*Glu;
R² is -NH₂ or -OH;
X1 is Ala, Aib, Pro or Gly;
X2 is Thr, Pro or Ser;
X3 is Aib, Bip, β,β-Dip, F5-Phe, Phe, PhG, Nle, homoPhe, homoTyr, *N*-MePhe, α-MePhe, α-Me-2F-Phe, Tyr, Trp, Tyr-OMe, 4I-Phe, 2F-Phe, 3F-Phe, 4F-Phe, 1-NaI, 2-NaI, Pro or di-β,β-MePhe;
X4 is Aib, Ala, Asp, Arg, Bip, Cha, β,β-Dip, Gln, F5-Phe, PhG, Nle, homoPhe, homoTyr, α-MePhe, α-Me-2F-Phe, Phe, Thr, Trp, Tyr-OMe, 4I-Phe, 2F-Phe, 3F-Phe, 4F-Phe, Tyr, 1-NaI, 2-NaI, Pro, di-β,β-MePhe, α-MeTyr or di-β,β-MeTyr;
X5 is Aib, Lys, D-pro, Pro or α-MeLys or di-β,β-MeLys;
X6 is Aib, Asp, Arg, Bip, Cha, Leu, Lys, 2Cl-Phe, 3Cl-Phe, 4Cl-Phe, PhG, homoPhe, 2Me-Phe, 3Me-Phe, 4Me-Phe, 2CF₃-Phe, 3CF₃-Phe, 4CF₃-Phe, β-Phe, β-MePhe, D-phe, 4I-Phe, 3I-Phe, 2F-Phe, β,β-Dip, β-Ala, Nle, Leu, F5-Phe, homoTyr, α-MePhe, α-Me-2F-Phe, Ser, Tyr, Trp, Tyr-OMe, 3F-Phe, 4F-Phe, Pro, 1-NaI, 2-NaI or di-β,β-MePhe; α-MeTyr, di-β,β-MeTyr, α-MeTrp or di-β,β-MeTrp;
X7 is Aib, Arg, Bip, Cha, β,β-Dip, F5-Phe, PhG, Phe, Tyr, homoPhe, homoTyr, α-MePhe, α-Me-2F-Phe, 2Me-Phe, 3Me-Phe, 4Me-Phe, Nle, Tyr-OMe, 4I-Phe, 1-NaI, 2-NaI, 2F-Phe, 3F-Phe, 4F-Phe, Pro, *N*-MeTrp, α-MeTrp, di-β,β-MeTrp, di-β,β-Me-Phe; α-MeTyr or di-β,β-MeTyr;
X8 is Aib, Ala, Arg, Asp, Glu, Nle, Pro, Ser, *N*-MeLeu, α-MeLeu, Val or α-MeVal;
X9 is Aib, Glu, Lys, Pro, α-MeVal or α-MeLeu;
X10 is Aib, Glu, Lys, Pro or α-MeLys;
X11 is Aib, Glu, Pro or Ser; and
X12 is Aib, Gly, Glu, Lys, Pro, α-MeArg or α-MeLys.

Further embodiments, features, and advantages of the embodiments, as well as the structure and operation of the various embodiments, are described in detail below with reference to accompanying drawings.

### BRIEF DESCRIPTIONS OF THE DRAWINGS

FIG 1. shows exemplary sites for amino acid substitution in glucagon-like peptide 1 (GLP-1). (SEQ ID NO:3)
FIGs. 2A-2C show neprilysin degradation of a GLP-1 comparator.
FIGs. 3A-3D show stability of synthetic GLP-1 proteins in accordance with embodiments described herein exposed to neprilysin.
FIGs. 4A-4D show stability of synthetic GLP-1 proteins in accordance with embodiments described herein after 240 hours exposed to neprilysin.
FIGs. 5A-5C show chymotrypsin degradation of a GLP-1 comparator.
FIGs. 6A-6D show stability of synthetic GLP-1 proteins in accordance with embodiments described herein exposed to chymotrypsin.
FIGs. 7A-7D show stability of synthetic GLP-1 proteins in accordance with embodiments described herein exposed to chymotrypsin.
FIGs. 8A-8C show trypsin degradation of a GLP-1 comparator.
FIGs. 9A-9C show stability of synthetic GLP-1 proteins in accordance with embodiments described herein exposed to trypsin.
FIGs. 10A-10C show stability of synthetic GLP-1 proteins in accordance with embodiments described herein exposed to trypsin.
FIGs. 11A-11B show serum degradation of a GLP-1 comparator.
FIGs. 12A-12B show stability of synthetic GLP-1 proteins in accordance with embodiments described herein exposed to serum.
FIGs. 13A-13D show stability of lipidated comparator and lipidated synthetic GLP-1 protein in accordance with embodiments described herein exposed to gastric fluid.
FIGs. 14A-14E show stability studies of a commercially available GLP-1 protein and a synthetic GLP-1 protein in accordance with embodiments described herein exposed to gastric fluid.
FIGs. 15A-15E show a zoomed spectrum demonstrating stability studies of a commercially available GLP-1 peptide and a synthetic GLP-1 peptide in accordance with embodiments described herein exposed to gastric fluid.

### DETAILED DESCRIPTION OF THE INVENTION

It should be appreciated that the particular implementations shown and described herein are examples and are not intended to otherwise limit the scope of the application in any way.

The published patents, patent applications, websites, company names, and scientific literature referred to herein are hereby incorporated by reference in their entirety to the same extent as if each was specifically and individually indicated to be incorporated by reference. Any conflict between any reference cited herein and the specific teachings of this specification shall be resolved in favor of the latter. Likewise, any conflict between an art-understood definition of a word or phrase and a definition of the word or phrase as specifically taught in this specification shall be resolved in favor of the latter.

As used in this specification, the singular forms "a," "an" and "the" specifically also encompass the plural forms of the terms to which they refer, unless the content clearly dictates otherwise. The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 20%.

Technical and scientific terms used herein have the meaning commonly understood by one of ordinary skill in the art to which the present application pertains, unless otherwise defined. Reference is made herein to various methodologies and materials known to those of skill in the art. Standard reference works setting forth the general principles of peptide synthesis include W.C.Chan and P.D.White., "Fmoc Solid Phase Peptide Synthesis: A Practical Approach", Oxford University Press, Oxford (2004).

The terms "polypeptide," "peptide," "protein," and "protein fragment" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymers.

The term "amino acid" refers to naturally occurring and synthetic amino acids, as well as amino acid analogs and amino acid mimetics that function similarly to the naturally occurring amino acids. Naturally occurring amino acids are those encoded by the genetic code, as well as those amino acids that are later modified, e.g., hydroxyproline, gamma-carboxyglutamate, and *O*-phosphoserine. Amino acid analogs refer to compounds that have the same basic chemical structure as a naturally occurring amino acid, e.g., an alpha carbon that is bound to a hydrogen, a carboxyl group, an amino group, and an R group, e.g., homoserine, norleucine, methionine sulfoxide, methionine methyl sulfonium. Such analogs can have modified R groups (e.g., norleucine) or modified peptide backbones, but retain the same basic chemical structure as a naturally occurring amino acid. Amino acid mimetics refer to chemical compounds that have a structure that is different from the general chemical structure of an amino acid, but that function similarly to a naturally occurring amino acid. The terms "amino acid" and "amino acid residue" are used interchangeably throughout.

The majority of chemical modifications intended to improve metabolic stability of peptides involve additional chemical manipulation following synthesis of the main peptide chain, e.g. lactamization, disulfide bridge closure, lipidation or PEGylation. Such modifications are often time-consuming and are likely to significantly increase the final cost of goods of any product.

As described herein, incorporation of alpha-methyl-functionalized amino acids directly into the main chain during standard peptide synthesis makes the methodologies described herein more straightforward and amenable to large-scale preparation. With regard to chemical synthesis of peptides which are naturally helical, such as the incretin class which includes GLP-1, glucagon, GIP, VIP, and secretin, as described herein, it is believed that the natural turn-inducing effect of alpha-methyl amino acids improves the crude yield of peptides during synthesis.

As described herein, alpha-methyl amino acids are strategically incorporated during synthesis of a synthetic peptide at a desired site(s). The modified amino acids allow the peptide to retain the native side-chain functionality, which is frequently crucial to the receptor potency of the peptide.

Provided herein are compositions and methods that address the natural enzymatic liability of peptides. By shielding susceptible sites (e.g., scissile bonds) with a site-specific incorporation of an alpha-methyl-functionalized amino acid, peptides are provided that demonstrate increased resistance to enzymatic degradation, while still maintaining substantially the same receptor potency and selectivity as a wild-type peptide.

### Synthetic Peptides Demonstrating Protease Resistance

In embodiments, a synthetic peptide comprising at least one substitution of an alpha-methyl functionalized amino acid for a native amino acid residue is provided. In other embodiments, a synthetic peptide comprising at least two substitutions of alpha-methyl functionalized amino acids for native amino acid residues is provided.

As described herein, "synthetic peptide" refers to a polymer of amino acid residues that has been generated by chemically coupling a carboxyl group or C-terminus of one amino acid to an amino group or *N*-terminus of another. Chemical peptide synthesis starts at the C-terminal end of the peptide and ends at the *N*-terminus. Various methods for peptide synthesis to generate synthetic peptides are well known in the art.

As described herein "alpha-methyl functionalized amino acids" refer to amino acids in which the first (alpha) carbon atom of the amino acid includes a methyl group (CH₃) substituent bound to the alpha carbon. Alpha-methyl functionalized amino acids include any of the twenty-one amino acids that include such a functionalization.

As described throughout, alpha-methyl functionalized amino acids can be substituted, i.e., can replace, any native amino acid in a peptide. The "native" amino acid refers to the amino acid that is present in the natural or wild-type peptide, which is to be substituted.

Substitution refers to the replacement of a native amino acid with an alpha-functionalized amino acid. During chemical synthesis of a synthetic peptide, the native amino acid can be readily replaced by an alpha functionalized amino acid.

While the synthetic peptides described herein can be of any length, i.e., any number of amino acids in length, suitably the synthetic peptides are on the order of about 5 amino acids to about 200 amino acids in length, suitably about 10 amino acids to about 150 amino acids in length, about 20 amino acids to about 100 amino acids in length, about 30 amino acids to about 75 amino acids in length, or about 20 amino acids, about 30 amino acids, about 40 amino acids, about 50 amino acids, about 60 amino acids, about 70 amino acids, about 80 amino acids, about 90 amino acids or about 100 amino acids in length.

As described throughout, the synthetic peptides described herein that contain one or more alpha-functionalized amino acids substituted for native amino acids maintain substantially the same receptor potency and selectivity as a corresponding synthetic peptide that does not comprise the substitutions. In some cases, the synthetic peptides contain two or more alpha-functionalzed amino acids substituted for the native amino acids.

The term "receptor potency" refers to the inverse of the half maximum (50%) effective concentration (EC₅₀) of the peptide. The EC₅₀ refers to the concentration of peptide that induces a biological response halfway between the baseline response and maximum response, after a specified exposure time, for a selected target of the peptide. Thus, peptides exhibiting a small value for EC₅₀ have a corresponding high receptor potency, while peptides exhibiting a large value for EC₅₀ have a corresponding low receptor potency - the more peptide required to induce a response related to a receptor, the less potent the peptide is for that receptor.

Methods for determining the receptor potency and EC₅₀ are known in the art and suitably involve determining stimulation of one or more cellular receptor responses. For example, suitable cell lines expressing GLP-1 receptor (GLP-1R), glucagon receptor (GCGR) or glucose-dependent insulinotropic peptide (gastric inhibitory polypeptide) receptor (GIPR) are generated by standard methods. Peptide activation of these various receptors results in downstream production of a cAMP second messenger which can be measured in a functional activity assay. From these measurements, EC₅₀ values are readily determined.

As described throughout, the synthetic peptides which comprise one or more substitutions of alpha-functionalized amino acids (also called "substituted peptides" herein) maintain "substantially the same" receptor potency as a corresponding synthetic peptide that does not comprise the substitutions. As used herein, "substantially the same" when referring to receptor potency, means that the substituted peptides exhibit suitably about 75% of the receptor potency when the substituted peptides are compared to the receptor potency of peptides that do not contain any substitutions, and rather, contain the original, unmodified, wild-type sequence, or other suitable comparator sequence (i.e. a control). In further embodiments, the substituted peptides exhibit suitably about 80% of the receptor potency, or about 85% of the receptor potency, or about 90% of the receptor potency, or about 91% of the receptor potency, or about 92% of the receptor potency, or about 93% of the receptor potency, or about 94% of the receptor potency, or about 95% of the receptor potency, or about 96% of the receptor potency, or about 97% of the receptor potency, or about 98% of the receptor potency, or about 99% of the receptor potency, or about 99.1% of the receptor potency, or about 99.2% of the receptor potency, or about 99.3% of the receptor potency, or about 99.4% of the receptor potency, or about 99.5% of the receptor potency, or about 99.6% of the receptor potency, or about 99.7% of the receptor potency, or about 99.8% of the receptor potency, or about 99.9% of the receptor potency, or suitably about 100% of the receptor potency, when the substituted peptides are compared to the receptor potency of peptides that do not contain any substitutions, and rather, contain the original, unmodified, wild-type sequence, or other suitable comparator sequence (i.e. a control).

As described throughout, the synthetic peptides which comprise one or more substitutions of alpha-functionalized amino acids also suitably maintain "substantially the same selectivity" as a corresponding synthetic peptide that does not comprise the substitutions. As used herein, "selectivity," refers to the ability of a peptide to bind its target (i.e., the agonist to which it is designed to bind) while not binding to other non-target proteins. Suitably the substituted peptides exhibit "substantially the same selectivity" and thus exhibit about 75% of the selectivity when the substituted peptides are compared to the receptor potency of peptides that do not contain any substitutions, and rather, contain the original, unmodified, wild-type sequence, or other suitable comparator sequence (i.e. a control). In further embodiments, the substituted peptides exhibit suitably about 80% of the selectivity, or about 85% of the selectivity, or about 90% of the selectivity, or about 91% of the selectivity, or about 92% of the selectivity, or about 93% of the selectivity, or about 94% of the selectivity, or about 95% of the selectivity, or about 96% of the selectivity, or about 97% of the selectivity, or about 98% of the selectivity, or about 99% of the selectivity, or about 99.1% of the selectivity, or about 99.2% of the selectivity, or about 99.3% of the selectivity, or about 99.4% of the selectivity, or about 99.5% of the selectivity, or about 99.6% of the selectivity, or about 99.7% of the selectivity, or about 99.8% of the selectivity, or about 99.9% of the selectivity, or suitably about 100% of the selectivity, when the substituted peptides are compared to the selectivity of peptides that do not contain any substitutions, and rather, contain the original, unmodified, wild-type sequence, or other suitable comparator sequence (i.e. a control).

Suitably, the alpha-methyl functionalized amino acids correspond to the substituted native amino acids in the wild-type protein. That is the amino acid in the original, wild-type peptide sequence is substituted with an alpha-methyl functionalized amino acid that has the same side chain. In other words, for example, Phe, Trp, Tyr, etc., are substituted with α-MePhe, α-MeTrp, α-MeTyr, respectively, etc.

In further embodiments, the alpha-methyl functionalized amino acids correspond to the same class as the substituted native amino acids. For example, aliphatic alpha-methyl functionalized amino acids are substituted for aliphatic native amino acids; hydroxyl alpha-methyl functionalized amino acids are substituted for hydroxyl native amino acids; sulfur-containing alpha-methyl functionalized amino acids are substituted for sulfur-containing native amino acids; cyclic alpha-methyl functionalized amino acids are substituted for cyclic native amino acids; aromatic alpha-methyl functionalized amino acids are substituted for aromatic native amino acids; basic alpha-methyl functionalized amino acids are substituted for basic native amino acids; and/or acidic alpha-methyl functionalized amino acids are substituted for acidic native amino acids.

In additional embodiments, the alpha-methyl functionalized amino acids do not correspond to the substituted native amino acids.

Commercial sources of alpha-methyl functionalized amino acids include, for example, Bachem AG, Switzerland.

In exemplary embodiments, at least one alpha-methyl functionalized amino acid in the synthetic peptides described herein is alpha-methyl phenylalanine.

In still further embodiments, at least one alpha-methyl functionalized amino acid in the synthetic peptides described herein is selected from alpha-methyl functionalized Histidine, alpha-methyl functionalized Alanine, alpha-methyl functionalized Isoleucine, alpha-methyl functionalized Arginine, alpha-methyl functionalized Leucine, alpha-methyl functionalized Asparagine, alpha-methyl functionalized Lysine, alpha-methyl functionalized Aspartic acid, alpha-methyl functionalized Methionine, alpha-methyl functionalized Cysteine, alpha-methyl functionalized Phenylalanine, alpha-methyl functionalized Glutamic acid, alpha-methyl functionalized Threonine, alpha-methyl functionalized Glutamine, alpha-methyl functionalized Tryptophan, alpha-methyl functionalized Glycine, alpha-methyl functionalized Valine, alpha-methyl functionalized Ornithine, alpha-methyl functionalized Proline, alpha-methyl functionalized Selenocysteine, alpha-methyl functionalized Serine and alpha-methyl functionalized Tyrosine.

As described throughout, the synthetic peptides described herein are substantially resistant to proteolytic degradation.

As used herein, "proteolytic degradation" means the breakdown of peptides into smaller peptides or even amino acids, generally caused by the hydrolysis of a peptide bond by enzymes.

The synthetic peptides provided throughout that are "substantially resistant" to proteolytic degradation indicates that at least about 50% of the synthetic peptide remains intact following exposure to an enzyme in conditions that the enzyme is generally active (i.e., suitable pH, temperature, other environmental conditions) for a defined period of time. Suitably, the synthetic peptides provided herein are substantially resistant to proteolytic degradation for a period of at least 4 hours, more suitably at least 8 hours, at least 12 hours, at least 24 hours, at least 36 hours, at least 48 hours, at least 72 hours, at least 96 hours, at least 120 hours, at least 144 hours, at least 168 hours, at least 192 hours, at least 216 hours, at least 240 hours, or about 36 hours to about 240 hours, about 48 hours to 240 hours, about 72 hours to about 240 hours, about 96 hours to about 240 hours, about 120 hours to about 240 hours, about 144 hours to about 240 hours, about 168 hours to about 240 hours, about 192 hours to about 240 hours, or about 216 hours to about 240 hours. In additional embodiments, at least about 80% of the synthetic peptide remains intact following exposure to an enzyme in conditions that the enzyme is generally active for a defined period of time, or more suitably at least about 60%, at least about 70%, at least about 80%, at least about 85%, at least about 90%, at least about 95%, at least about 96%, at least about 97%, at least about 98%, at least about 99%, at least about 99.1%, at least about 99.2%, at least about 99.3%, at least about 99.4%, at least about 99.5%, at least about 99.6%, at least about 99.7%, at least about 99.8%, at least about 99.9%, or at least about 100% of the synthetic peptide remains intact following exposure to an enzyme in conditions that the enzyme is generally active for a defined period of time.

The synthetic peptides provided are suitably substantially resistant to proteolytic degradation by one or more enzymes found in a mammalian body, suitably the human body. For example, the synthetic peptides are suitably resistant to proteolytic degradation by one or more of dipeptidyl peptidase-IV (DPP-IV), neprilysin, chymotrypsin, plasmin, thrombin, kallikrein, trypsin, elastase and pepsin. In suitable embodiments, the synthetic peptides are resistant to proteolytic degradation by to two or more, three or more, four or more, five or more, six or more, seven or more, or suitably all of the recited enzymes. The synthetic peptides described herein can also substantially resistant to proteolytic degradation by other enzymes known in the art. In embodiments, the synthetic peptides described herein are substantially resistant to proteolytic degradation by digestive (gastric) enzymes and/or enzymes in the blood/serum.

In embodiments, the synthetic peptides described herein are substantially resistant to proteolytic degradation by DPP-IV and neprilysin. In embodiments, the synthetic peptides described herein are substantially resistant to proteolytic degradation by pepsin, trypsin, chymotrypsin, and elastase. In embodiments, the synthetic peptides described herein are substantially resistant to proteolytic degradation by plasmin, thrombin and kallikrein. In embodiments, the synthetic peptides described herein are substantially resistant to proteolytic degradation by pepsin, trypsin and chymotrypsin. In embodiments, the synthetic peptides described herein are substantially resistant to proteolytic degradation by pepsin and trypsin.

As described herein, including in the methods provided throughout, substitution of alpha-functionalized amino acids for native amino acids suitably occurs at native amino acid residues that are sites susceptible to proteolytic cleavage. That is, the amino acid residues that are substituted are determined to be sites where proteolytic enzymes are active in cleaving peptide bonds in the natural (i.e., wild-type) peptides. Methods for determining sites of proteolytic cleavage are well known in the art and described herein.

Any class of peptide can be prepared according to the methods provided herein to yield synthetic peptides having the recited characteristics.

In exemplary embodiments, the synthetic peptides are incretin class peptides. Exemplary synthetic incretin class peptides that can be prepared as described herein include, but are not limited to, glucagon-like peptide 1 (GLP-1), a glucose-dependent insulinotropic peptide (GIP), an exenatide peptide, plus glucagon, secretins, tenomodulin, oxyntomodulin or vasoactive intestinal peptide (VIP).

Additional classes of peptides can be prepared as described herein.

In embodiments, the synthetic peptide described herein is a GLP-1 peptide. In further embodiments, the synthetic peptide described herein is insulin.

Sequences for the native (wild type) peptides of the various peptides and classes of peptides described herein that can be prepared to yield synthetic peptides having the recited characteristics are well known in the art.

The native amino acid sequence for GPL-1 is known in the art as set forth below:
HAEGTFTSDVSSYLEGQAAKEFIAWLVKGR (SEQ ID NO:1).

In embodiments, synthetic GLP-1 peptides are provided comprising at least three substitutions of alpha-methyl functionalized amino acids for native amino acid residues. As described throughout, suitably the synthetic GLP-1 peptide maintains substantially the same receptor potency and selectivity as a corresponding synthetic GLP-1 peptide that does not comprise the substitutions.

In embodiments, the at least three alpha-methyl functionalized amino acids are substituted for the corresponding native amino acid residues. That is, as described herein, the amino acid in the native protein is substituted with the same, corresponding alpha-methyl functionalized amino acid.

In additional embodiments, the three alpha-methyl functionalized amino acids are alpha-methyl phenylalanine. In such embodiments, it is not necessary that the native amino acids that are being substituted for by the alpha-methyl functionalized phenylalanine are themselves phenylalanine. Rather, as described herein, simply by replacing a native aromatic amino acid with an alpha-methyl functionalized amino acid from the same class, i.e., an aromatic amino acid, the synthetic peptides described herein have been found to exhibit the desired characteristics of maintained receptor potency and selectivity as well as increased stability.

In additional embodiments, the synthetic peptides described herein can further comprise modification by lipidation, including carboxyl- or amino- terminal lipidation, or main-chain lipidation. Methods of preparing synthetic peptides with such a lipidation are known in the art. It has been determined that, in combination with the embodiments described herein where native amino acids are substituted for by alpha-methyl functionalized amino acids, that C-terminal lipidation provides additional stability, particularly during exposure to serum and gastric fluid.

Suitably, the synthetic GLP-1 peptides provided herein comprise four alpha-methyl functionalized amino acids. In embodiments, the four alpha-methyl functionalized amino acids are substituted for corresponding amino acids. In exemplary embodiments, the four alpha-methyl functionalized amino acids are substituted at positions Phe6, Try13, Phe22 and Trp25, and in further embodiments, the four alpha-methyl functionalized amino acids are alpha-methyl phenylalanine substituted at positions Phe6, Try13, Phe22 and Trp25.

In further embodiments, the synthetic GLP-1 peptides provided herein comprise six alpha-methyl functionalized amino acids. In embodiments, the six alpha-methyl functionalized amino acids are substituted for corresponding amino acids. In exemplary embodiments, the six alpha-methyl functionalized amino acids are substituted at positions Phe6, Try13, Lys20, Phe22, Trp25 and Lys28, and in further embodiments, the six alpha-methyl functionalized amino acids are four alpha-methyl phenylalanines substituted at positions Phe6, Try13, Phe22 and Trp25, and two alpha-methyl lysines substituted at positions Lys20 and Lys28.

In suitable embodiments, the GLP-1 synthetic peptides described herein suitably further comprise an aminoisobutyric acid substitution at position 2 (Aib2). In still further embodiments, the GLP-1 synthetic peptides described herein suitably further comprise a Serine substitution for Threonine at position 5 (Thr5Ser; T5S). In still further embodiments, the GLP-1 synthetic peptides described herein suitably further comprise a Valine substitution for Leucine at position 26 (Leu26Val; L26V).

In embodiments, synthetic GLP-1 peptides described herein are substantially resistant to proteolytic degradation, including but not limited to, degradation by one or more of DPP-IV, neprilysin, chymotrypsin, plasmin, thrombin, kallikrein, trypsin, elastase and pepsin.

In additional embodiments, provided herein are GLP-1 peptides comprising the following amino acid sequence, in order:

R¹-His-X1-Glu-Gly-X2-X3-Thr-Ser-Asp-Val-Ser-Ser-X4-Leu-Glu-Gly-Gln-Ala-Ala-X5-Glu-X6-Ile-Ala-X7-X8-X9-X10-X11-X12-R² (SEQ ID NO:2)

, wherein:
R¹ is Hy, Ac or *p*Glu;
R² is -NH₂ or -OH;
X1 is Ala, Aib, Pro or Gly;
X2 is Thr, Pro or Ser;
X3 is Aib, Bip, β,β-Dip, F5-Phe, Phe, PhG, Nle, homoPhe, homoTyr, *N*-MePhe, α-MePhe, α-Me-2F-Phe, Tyr, Trp, Tyr-OMe, 4I-Phe, 2F-Phe, 3F-Phe, 4F-Phe, 1-NaI, 2-NaI, Pro or di-β,β-Me-Phe;
X4 is Aib, Ala, Asp, Arg, Bip, Cha, β,β-Dip, Gln, F5-Phe, PhG, Nle, homoPhe, homoTyr, α-MePhe, α-Me-2F-Phe, Phe, Thr, Trp, Tyr-OMe, 4I-Phe, 2F-Phe, 3F-Phe, 4F-Phe, Tyr, 1-NaI, 2-NaI, Pro or di-β,β-Me-Phe;
X5 is Aib, Lys, D-pro, Pro or α-MeLys;
X6 is Aib, Asp, Arg, Bip, Cha, Leu, Lys, 2Cl-Phe, 3Cl-Phe, 4Cl-Phe, PhG, homoPhe, 2Me-Phe, 3Me-Phe, 4Me-Phe, 2CF₃-Phe, 3CF₃-Phe, 4CF₃-Phe, β-Phe, β-MePhe, D-phe, 4I-Phe, 3I-Phe, 2F-Phe, β,β-Dip, β-Ala, Nle, Leu, F5-Phe, homoTyr, α-MePhe, α-Me-2F-Phe, Ser, Tyr, Trp, Tyr-OMe, 3F-Phe, 4F-Phe, Pro, 1-NaI, 2-NaI or di-β,β-Me-Phe;
X7 is Aib, Arg, Bip, Cha, β,β-Dip, F5-Phe, PhG, Phe, Tyr, homoPhe, homoTyr, α-MePhe, α-Me-2F-Phe, 2Me-Phe, 3Me-Phe, 4Me-Phe, Nle, Tyr-OMe, 4I-Phe, 1-NaI, 2-NaI, 2F-Phe, 3F-Phe, 4F-Phe, Pro, *N*-MeTrp, α-MeTrp or di-β,β-Me-Phe;
X8 is Aib, Ala, Arg, Asp, Glu, Nle, Pro, Ser, i*N*-MeLeu, α-MeLeu or Val;
X9 is Aib, Glu, Lys, α-MeVal or Pro;
X10 is Aib, Glu, α-MeLys or Pro;
X11 is Aib, Glu, Pro or Ser; and
X12 is Aib, Gly, Glu, Pro or α-MeArg.

Suitably, the GLP-1 peptides consist of the amino acid sequence set forth in SEQ ID NO:2, i.e., consist only of the recited amino acids in the complete sequence, and in the recited order, as set forth in SEQ ID NO:2.

### Methods of Preparing Synthetic Peptides

Also provided are methods of preparing synthetic peptides.

In some embodiments, the methods suitably comprise identifying at least one native amino acid residue in the peptide for substitution. In other embodiments, the methods suitably comprise identifying at least two native amino acid residues in the peptide for substitution. Alpha-methyl functionalized amino acids are then substituted for the identified native amino acid residues.

As described throughout, the synthetic peptides prepared by the methods provided herein suitably maintain substantially the same receptor potency and selectivity as a corresponding synthetic peptide that does not comprise the substitutions. In addition, the synthetic peptides prepared according to the methods described herein are also substantially resistant to proteolytic degradation.

Suitably in the methods provided herein the substituted alpha-methyl functionalized amino acids correspond to the substituted native amino acid residues, and in additional embodiments, the substituted alpha-methyl functionalized amino acids correspond to the same class as the substituted native amino acid residues.

In further embodiments, the substituted alpha-methyl functionalized amino acids are alpha-methyl phenylalanine. In exemplary embodiments, alpha-methyl phenylalanine is substituted for corresponding native amino acids, though in further embodiments of the methods, the alpha-methyl phenylalanine do not have to correspond to the same native amino acids for which the substitution is occurring.

In suitable embodiments, the synthetic peptides prepared according to the methods described herein are substantially resistant to one or more of DPP-IV, neprilysin, chymotrypsin, plasmin, thrombin, kallikrein, trypsin, elastase and pepsin degradation.

In embodiments, synthetic peptides are prepared as C-terminal carboxamides on NOVASYN® TGR resin. Amino acids (both natural and unnatural) are suitably coupled at ambient temperature using HCTU/DIPEA in NMP, capping residual functionality with a solution of acetic anhydride and pyridine. Fmoc is suitably deblocked in using piperidine in DMF at ambient temperature.

As described herein, identifying at least one native amino acid residue in the peptide for substitution suitably comprises identifying amino acids at sites susceptible to enzymatic cleavage. Exemplary methods of identifying amino acids at sites susceptible to enzymatic cleavage are well known in the art. In embodiments, methods of identifying amino acids at sites susceptible to enzymatic cleavage suitably comprise exposing a natural peptide (i.e., a wild-type peptide) to a single enzyme under conditions in which the enzyme is active (e.g., suitable pH, buffer conditions, temperature, etc.) for a pre-determined amount of time and measuring the enzymatic degradation products of the peptide. Exemplary methods for measuring the enzymatic degradation products include, for example, reverse-phase liquid chromatography-mass spectrometry.

Suitably, peptide solutions are added to solutions of a desired protease. The peptide and enzyme are the co-incubated, suitably at about 37°C. Aliquots of the incubated peptide-enzyme mixture are withdrawn periodically, quenched to arrest proteolytic activity, and analyzed by liquid chromatography-mass spectrometry (LC/MS). Analytes are suitably detected by both UV absorption (e.g., at 210 nm) and by ionization using a mass detector (ESI+ mode). Peptidic species (fragments) deriving from enzymatic cleavage of peptides are analyzed post-process, and their molecular masses are used to identify the precise cleavage position (highlighting the scissile bond in each case).

In embodiments, the methods described herein are suitably used to prepare any class of peptide having the recited characteristics.

In exemplary embodiments, the methods are used to prepare are incretin class peptides. Exemplary synthetic incretin class peptides that can be prepared as described herein include, but are not limited to, glucagon-like peptide 1 (GLP-1), a glucose-dependent insulinotropic peptide (GIP), an exenatide peptide, plus glucagon, secretins, tenomodulin and oxyntomodulin.

Additional classes of peptides can be prepared as described herein.

In embodiments, the methods are used to prepare synthetic GLP-1 peptides. In further embodiments, the methods are used to prepare synthetic insulin.

In further embodiments, methods of preparing a proteolytically stable peptide are provided. Suitably, such methods comprise exposing a peptide to one or more proteases, identifying at least two native amino acid residues which are sites susceptible to proteolytic cleavage, and substituting alpha-methyl functionalized amino acids for the identified amino acid residues.

As described throughout, suitably such methods provide a synthetic peptide that maintains substantially the same receptor potency and selectivity as a corresponding synthetic peptide that does not comprise the substitution(s). In further embodiments, the methods also provide a synthetic peptide that is substantially resistant to proteolytic degradation.

Suitably in the methods provided herein, the substituted alpha-methyl functionalized amino acids correspond to the substituted native amino acid residues, and in additional embodiments, the substituted alpha-methyl functionalized amino acids correspond to the same class as the substituted native amino acid residues.

In still further embodiments, the substituted alpha-methyl functionalized amino acids are selected from alpha-methyl functionalized Histidine, alpha-methyl functionalized Alanine, alpha-methyl functionalized Isoleucine, alpha-methyl functionalized Arginine, alpha-methyl functionalized Leucine, alpha-methyl functionalized Asparagine, alpha-methyl functionalized Lysine, alpha-methyl functionalized Aspartic acid, alpha-methyl functionalized Methionine, alpha-methyl functionalized Cysteine, alpha-methyl functionalized Phenylalanine, alpha-methyl functionalized Glutamic acid, alpha-methyl functionalized Threonine, alpha-methyl functionalized Glutamine, alpha-methyl functionalized Tryptophan, alpha-methyl functionalized Glycine, alpha-methyl functionalized Valine, alpha-methyl functionalized Ornithine, alpha-methyl functionalized Proline, alpha-methyl functionalized Selenocysteine, alpha-methyl functionalized Serine and alpha-methyl functionalized Tyrosine.

In further embodiments, the substituted alpha-methyl functionalized amino acids are alpha-methyl phenylalanine and/or alpha-methyl lysine. In exemplary embodiments, alpha-methyl phenylalanine and/or alpha-methyl lysine are substituted for corresponding native amino acids, though in further embodiments of the methods, the alpha-methyl phenylalanine and/or alpha-methyl lysine do not have to correspond to the same native amino acids for which the substitution is occurring.

In suitable embodiments, the synthetic peptides prepared according to the methods described herein are substantially resistant to one or more of DPP-IV, neprilysin, chymotrypsin, plasmin, thrombin, kallikrein, trypsin, elastase and pepsin degradation.

In embodiments, the methods described herein are suitably used to prepare any class of peptide having the recited characteristics.

In exemplary embodiments, the methods are used to prepare are incretin class peptides. Exemplary synthetic incretin class peptides that can be prepared as described herein include, but are not limited to, glucagon-like peptide 1 (GLP-1), a glucose-dependent insulinotropic peptide (GIP), an exenatide peptide, plus glucagon, secretins, tenomodulin and oxyntomodulin.

Additional classes of peptides can be prepared as described herein.

In embodiments, the methods are used to prepare synthetic GLP-1 peptides. In further embodiments, the methods are used to prepare synthetic insulin.

### Formulations Comprising Synthetic Peptides

Also provided are formulations (or pharmaceutical compositions) comprising a synthetic peptide described herein. Suitably such formulations comprise a synthetic peptide as described herein and a carrier. Such formulations can be readily administered in the various methods described throughout. In some embodiments, the formulation comprises a pharmaceutically acceptable carrier.

The term "pharmaceutically acceptable carrier" means one or more non-toxic materials that do not interfere with the effectiveness of the biological activity of the synthetic peptides. Such preparations may routinely contain salts, buffering agents, preservatives, compatible carriers, and optionally other therapeutic agents. Formulations may also routinely contain compatible solid or liquid fillers, diluents or encapsulating substances which are suitable for administration into a human. The term "carrier" denotes an organic or inorganic ingredient, natural or synthetic, with which the synthetic peptide is combined to facilitate the application.

Formulations as described herein may be formulated for a particular dosage. Dosage regimens may be adjusted to provide the optimum desired response. For example, a single bolus may be administered, several divided doses may be administered over time or the dose may be proportionally reduced or increased as indicated by the therapeutic situation. It is especially advantageous to formulate parenteral compositions in dosage unit forms for ease of administration and uniformity of dosage. Dosage unit forms as used herein refers to physically discrete units suited as unitary dosages for the subjects to be treated; each unit contains a predetermined quantity of a synthetic peptide calculated to produce the desired therapeutic effect in association with the required pharmaceutical carrier. The specification for the dosage unit forms are dictated by, and directly dependent on, (a) the unique characteristics of the synthetic peptide and the particular therapeutic effect to be achieved, and (b) the limitations inherent in the art of compounding such a synthetic peptide.

Formulations described herein can be formulated for particular routes of administration, such as oral, nasal, pulmonary, topical (including buccal and sublingual), rectal, vaginal and/or parenteral administration. The formulations may conveniently be presented in unit dosage form and may be prepared by any methods known in the art of pharmacy. The amount of synthetic peptide which can be combined with a carrier material to produce a single dosage form will vary depending upon the subject being treated, and the particular mode of administration. The amount of synthetic peptide which can be combined with a carrier material to produce a single dosage form will generally be that amount of the composition which produces a therapeutic effect.

### Methods of Treatment Utilizing Synthetic Peptides

Also provided herein are methods of treating a patient comprising administering a synthetic peptide, e.g., the formulations, described herein to a patient in need thereof.

Suitably subjects that can be administered the synthetic peptides in the various methods described herein are mammals, such as for example, humans, dogs, cats, primates, cattle, sheep, horses, pigs, etc.

Exemplary methods by which the synthetic peptides can be administered to the subject in any of the various methods described herein include, but are not limited to, intravenous (IV), intratumoral (IT), intralesional (IL), aerosal, percutaneous, oral, endoscopic, topical, intramuscular (IM), intradermal (ID), intraocular (IO), intraperitoneal (IP), transdermal (TD), intranasal (IN), intracereberal (IC), intraorgan (e.g. intrahepatic), slow release implant, or subcutaneous administration, or via administration using an osmotic or mechanical pump.

Suitably, the synthetic peptides are administered as soon as possible after a suitable diagnosis, e.g., within hours or days. The duration and amount of synthetic peptide to be administered are readily determined by those of ordinary skill in the art and generally depend on the type of peptide and disease or disorder being treated.

As described herein, suitably the various methods are carried out on mammalian subject that are humans, including adults of any age and children.

In embodiments, the methods of treatment comprise treating a patient diagnosed with diabetes comprising administering a therapeutically effective amount of a suitable synthetic peptide as described herein, suitably a synthetic GLP-1 peptide as described herein.

As used herein, the term "therapeutically effective amount" refers to the amount of a synthetic peptide, or formulation, that is sufficient to reduce the severity of a disease or disorder (or one or more symptoms thereof), ameliorate one or more symptoms of such a disease or disorder, prevent the advancement of such a disease or disorder, cause regression of such a disease or disorder, or enhance or improve the therapeutic effect(s) of another therapy. In some embodiments, the therapeutically effective amount cannot be specified in advance and can be determined by a caregiver, for example, by a physician or other healthcare provider, using various means, for example, dose titration. Appropriate therapeutically effective amounts can also be determined by routine experimentation using, for example, animal models.

In embodiments, methods are provided of treating a patient diagnosed with diabetes comprising administering a therapeutically effective amount of synthetic insulin to a patient.

As described herein, suitably the methods of administration of the synthetic peptides or formulations described herein are delivered orally. As described herein, the synthetic peptides are substantially resistant to proteolytic degradation, i.e., degradation by enzymes in the stomach following oral administration.

It will be readily apparent to one of ordinary skill in the relevant arts that other suitable modifications and adaptations to the methods and applications described herein can be made without departing from the scope of any of the embodiments. The following examples are included herewith for purposes of illustration only and are not intended to be limiting.

### EXAMPLES

### Example 1: Chemical Synthesis and Testing of Proteolytic-Resistant Peptides

### 1. Introduction

The following provides exemplary methods for preparing proteolytic-resistant peptides as described herein.

### 2. Abbreviations

Boc, *tert*-butyloxycarbonyl; DIPEA, *N,N-*diisopropylethylamine; DMF, *N,N-*dimethylformamide; DMSO, dimethylsulfoxide; ESI, electrospray ionisation; Fmoc, 9-fluorenylmethyloxycarbonyl; GIP, gastric inhibitory polypeptide; GLP-1, glucagon-like peptide 1; HCTU, *O*-(1H-6-chlorobenzotriazole-1-yl)-1,1,3,3-tetramethyluronium hexafluorophosphate; RP-HPLC, reversed-phase high-performance liquid chromatography; EC₅₀, half maximal (50%) effective concentration; LC/MS, liquid chromatography-coupled mass spectrometry; MeCN, acetonitrile; NMP, N-methylpyrrolidinone; Pbf, 2,2,4,6,7-pentamethyldihydrobenzofuran-5-sulfonyl; PBS, phosphate buffered saline; ^{t}Bu, tertiary-butyl; TFA, trifluoroacetic acid; TIS, triisopropylsilane; Tris, Tris(hydroxymethyl)aminomethane; Trt, triphenylmethyl; UV, ultraviolet.

### 3. Experimental

### 3.1 Peptide Synthesis

### 3.1.1 Materials

*N*-α-Fmoc-*L*-amino acids were obtained from Bachem AG, Switzerland. Unusuaal amino acids were obtained from Iris Biotech AG, Germany or prepared by Pharmaron, China. NOVASYN® TGR (TentaGel Rink) and NOVASYN ® TGA (TentaGel Wang) synthesis resins were obtained from Novabiochem, Merck Biosciences, Darmstadt, Germany. All peptides were prepared by automated synthesis (PTI Prelude) using the Fmoc/^{t}Bu protocol. Asparagine (Asn) and glutamine (Gln) were incorporated as their sidechain trityl (Trt) derivatives. Tryptophan (Trp) and lysine (Lys) were incorporated as their sidechain Boc derivatives. Serine (Ser), threonine (Thr) and tyrosine (Tyr) were incorporated as sidechain ^{t}Bu ethers, and aspartate (Asp) and glutamate (Glu) as their sidechain O^{t}Bu esters. Arginine (Arg) was incorporated as the sidechain Pbf derivative. Synthesis reagents were obtained from Sigma-Aldrich, Dorset, United Kingdom. Solvents were obtained from Merck, Darmstadt, Germany at the highest grade available and used without further purification.

### 3.1.2 General procedure for chemical synthesis of peptides containing α-methyl amino acids

Unless otherwise stated, all peptides were prepared as C-terminal carboxamides on NOVASYN® TGR resin (initial substitution 0.24 mmole/g). All amino acids (both natural and unnatural) were coupled at ambient temperature using HCTU/DIPEA in NMP, capping residual functionality with a solution of acetic anhydride and pyridine. Fmoc was deblocked in using piperidine in DMF (20% v/v) at ambient temperature.

### 3.1.3 Cleavage and purification of linear peptides

Crude peptides were cleaved from the resin support by treatment with a cocktail of TFA (95% v/v), TIPS (2.5% v/v), water (2.5% v/v) at ambient temperature with agitation. Cleavage aliquots were combined, concentrated by rotary evaporation and precipitated by addition of cold diethyl ether, isolating solids by centrifugation. Crude peptides were dried under a flow of dry nitrogen, reconstituted in 20% MeCN/water (v/v) and filtered. Crude peptides were chromatographed using an Agilent Polaris C8-A stationary phase (21.2 x 250 mm, 5 micron) eluting with a linear solvent gradient from 10% to 70% MeCN (0.1% TFA v/v) in water (0.1% TFA v/v) over 30 minutes using a Varian SD-1 Prep Star binary pump system, monitoring by UV absorption at 210 nm. The desired peptide-containing fractions were pooled, frozen (dry-ice/acetone) and lyophilized.

### 3.1.4 Peptide analysis and characterization (post synthesis)

Purified peptides were characterized by single quadrupolar LC/MS using a Waters Mass Lynx 3100 platform. Analytes were chromatographed by elution on a Waters X-Bridge C18 stationary phase (4.6 x 100 mm, 3 micron) using a linear binary gradient of 10-90% MeCN (0.1% TFA v/v) in water (0.1% TFA v/v) over 10 minutes at 1.5 mL min⁻¹ at ambient temperature. Analytes were detected by both UV absorption at 210 nm and ionization using a Waters 3100 mass detector (ESI+ mode), verifying molecular masses against calculated theoretical values. Analytical RP-HPLC spectra were recorded using an Agilent 1260 Infinity system. Analytes were chromatographed by elution on an Agilent Polaris C8-A stationary phase (4.6 x 100 mm, 3 micron) at 1.5 mL min⁻¹ a linear binary gradient of 10-90% MeCN (0.1% TFA v/v) in water (0.1% TFA v/v) over 15 minutes at 40 °C.

### 4 Enzymatic Cleavage Studies

### 4.1 Evaluating proteolytic resistance of peptides containing α-methyl residues

The following commercially available purified proteases were evaluated for their ability to cleave wild-type incretins and modified incretins containing α-methyl amino acids at known liable sites.

**Table 1: Examples of commercially available purified proteases**

| **Protease** | **Family** | **Cleavage Specificity** | **Notes** |
|---|---|---|---|
| Neprilysin | Zinc metalloprotease | Amino side of Tyr, Phe, Trp | R&D Systems: 1182-ZNC-010 |
| Pepsin | Aspartate protease | Amino side of Tyr, Phe, Trp, Leu | Sigma: P7012 |
| | | | M.W. 34,620 Da, -500 units/mg |
| Trypsin | Serine Protease | Carboxyl side of Arg and Lys | Sigma: P7409 (Type II-S) |
| | | | M.W. 23,800 Da, -1500 units/mg |
| Chymotrypsin | Serine Protease | Carboxyl side of Tyr, Phe, Trp, Leu | R&D Systems: 6907-SE-010 |

**Neutral endopeptidase (Neprilysin):** 1.0 µg *rh*NEP was reconstituted in 900µL of an assay buffer comprising: 50 mM Tris, 50mM NaCl, 50mM NaHCO₃, adjusting to pH 8.3 using NaOH (1.0 M).

**Pepsin:** 1.0 mg of lyophilized pepsin from porcine gastric mucosa was reconstituted in 900 µL of the following assay buffer: 10 mM HCl affording a 0.4% (w/v) solution at pH 2.0.

**Trypsin:** A solution of 1 mg/mL lyophilized trypsin from porcine pancreas was reconstituted in the following assay buffer: 50 mM Tris, 10 mM CaCl₂, 150 mM NaCl, 1 mM HCl, adjusting to pH 7.8.

**Chymotrypsin:** 1.0 µg rhCTRC was reconstituted in 900 µL of the following assay buffer: 50 mM Tris, 10 mM CaCl₂, 150 mM NaCl, 1 mM HCl, adjusting to pH 7.8.

### 4.1.2 Procedure

Peptides for evaluation were prepared to a concentration of 1.0 mg/mL solutions in either pure water, sterile saline for injection (0.9% w/v NaCl/water) or IX PBS (Dulbecco). 100µL (100 µg/mL peptide) of these solutions was added to 900µL of each protease solution. Additional experiments were performed examining protein degradation during exposure to serum and gastric fluid. For serum studies, peptides were incubated with 50% female Sprague-Dawley strain rat serum (SD rat serum). For gastric fluid studies, peptides were incubated 1:1 (volume: volume), fresh rat gastric fluid.

The peptide and enzyme (or serum or gastric fluid) were co-incubated in a temperature regulated water bath at 37°C for the duration of the experiment. During each experiment 100µL aliquots (10 µg peptide) of the incubated peptide-enzyme mixture were withdrawn periodically, quenched by addition of an equal volume of 5% TFA (v/v) in 1:1 water/acetonitrile to arrest proteolytic activity, and analyzed by liquid chromatography-mass spectrometry (LC/MS): Agilent Polaris C8-A column (4.6 x 100 mm, 3 micron) using a linear binary gradient of 10-90% MeCN (0.1% TFA v/v) in water (0.1% TFA v/v) over 30 minutes at 1.5 mL min⁻¹ at ambient temperature. Analytes were detected by both UV absorption at 210 nm and ionization using a Waters 3100 mass detector (ESI+ mode). New peptidic species (fragments) derived from enzymatic cleavage of peptides were analyzed post-process, and their molecular masses were used to identify the precise cleavage position (highlighting the scissile bond in each case).

The biological activities/receptor potencies of the synthetic GLP-1 peptides described herein are suitably tested for biological activity, e.g., stimulation of one or more cellular receptor responses. Stable cell lines expressing human, mouse, rat, or dog GLP-1 receptor (GLP-1R), glucagon receptor (GCGR) or glucose-dependent insulinotropic peptide (gastric inhibitory polypeptide) receptor (GIPR) are generated in HEK293 cells or CHO cells by standard methods. Peptide activation of these various receptors results in downstream accumulation of cAMP second messenger which can be measured in a functional activity assay.

cAMP assays were performed using "assay buffer": Assay Buffer: 0.1% BSA (Sigma # A3059) in HBSS (Sigma # H8264) with 25mM HEPES, pH 7.4 and containing 0.5mM IBMX (Sigma # 17018).

Low protein binding 384-well plates (Greiner # 781280) are used to perform eleven 1 in 5 serial dilutions of test samples which are made in assay buffer. All sample dilutions are made in duplicate.

A frozen cryo-vial of cells expressing the receptor of interest is thawed rapidly in a water-bath, transferred to pre-warmed assay buffer and spun at 240xg for 5 minutes. Cells are re-suspended in assay buffer at a batch-dependent optimized concentration (e.g. hGCGR cells at 2x10⁵ cells/ml, hGLP-1R and hGIPR cells at 1x10⁵ cells /ml).

From the dilution plate, a 5µL replica is stamped onto a black shallow-well u-bottom 384-well plate (Corning # 3676). To this, 5µL cell suspension is added and the plates incubated at room temperature for 30 minutes.

cAMP levels are measured using a commercially available cAMP dynamic 2 HTRF kit (Cisbio, Cat # 62AM4PEJ), following the two step protocol as per manufacturer's recommendations. In brief; anti-cAMP cryptate (donor fluorophore) and cAMP-d2 (acceptor fluorophore) are made up separately by diluting each 1/20 in conjugate & lysis buffer provided in the kit. 5µL anti-cAMP cryptate is added to all wells of the assay plate, and 5µL cAMP-d2 is added to all wells except non-specific binding (NSB) wells, to which conjugate and lysis buffer are added. Plates are incubated at room temperature for one hour and then read on an Envision (Perkin Elmer) using excitation wavelength of 320nm and emission wavelengths of 620nm & 665nm. EC₅₀ values of the synthetic peptides determined in cAMP assays are then determined.

In additional experiments for determining biological activity/receptor potency, CHO cells with stable recombinant expression of the human, mouse or rat GCGR or GLP-1 receptor are cultured in assay buffer as above). Cryopreserved cell stocks are prepared in 1x cell freezing medium-DMSO serum free (Sigma Aldrich) at either 1x10⁷ or 2x10⁷/vial and stored at -80°C. Cells are rapidly thawed at 37°C and then diluted into assay buffer (buffer as above) containing serum albumin at 4.4, 3.2 and 3.2% for human, rat, and mouse serum albumin respectively. Peptides are serially diluted in 100% DMSO and then diluted 100 fold into assay buffer as above containing serum albumin at stated final concentration. Diluted peptides are then transferred into 384 black shallow well microtitre assay plates. Cells are added to the assay plates and incubated for 30 min at room temperature. Following incubation the assay is stopped and cAMP levels measured using the HTRF® dynamic d2 cAMP assay kit available from CisBio Bioassays, as per the manufacturer's guidelines. Plates are read on Perkin Elmer ENVISION® fluorescence plate readers. Human and rat serum albumin are purchased from Sigma Aldrich and mouse serum albumin from Equitech Bio Ltd.

Data is transformed to % Delta F as described in the manufacturer's guidelines and analyzed by 4-parameter logistic fit to determine EC₅₀ values. EC₅₀ values determined are dependent on both the potency of the peptides tested at the GLP-1 and glucagon receptors in the recombinant cell lines and on the affinity of the peptide for serum albumin, which determines the amount of free peptide. Association with serum albumin increases the EC₅₀ value obtained. The fraction of free peptide at plasma concentrations of albumin and the EC₅₀ at 0% serum albumin (SA) can be calculated based on the variation in cAMP generation with the SA concentration. To compare the balance of activities at the GLP-1R and GCGR between different peptides and across different conditions, these can be correlated, where the EC₅₀'s are related to those of comparator peptides.

The biological activities/receptor potencies of the synthetic GLP-1 peptides described herein are suitably tested for biological activity, e.g., stimulation of one or more cellular receptor responses. Stable cell lines expressing human, mouse, rat, or dog GLP-1 receptor (GLP-1R), glucagon receptor (GCGR) or glucose-dependent insulinotropic peptide (gastric inhibitory polypeptide) receptor (GIPR) are generated in HEK293s or CHO cells by standard methods. Peptide activation of these various receptors results in downstream production of cAMP second messenger which can be measured in a functional activity assay.

cAMP assays were performed using "assay medium":
Assay Medium: 10% FBS in DMEM (Gibco # 41966), containing 0.5mM IBMX (Sigma # 17018).

Low protein binding 384-well plates (Greiner # 781280) are used to perform eleven 1 in 5 serial dilutions of test samples which are made in assay medium. All sample dilutions are made in duplicate.

A frozen cryo-vial of cells expressing the receptor of interest is thawed rapidly in a water-bath, transferred to pre-warmed assay media and spun at 240xg for 5 minutes. Cells are re-suspended in assay media at an optimized concentration (e.g. hGCGR cells at 1x10⁵ cells/ml, hGLP-1R and hGIPR cells at 0.5x10⁵ cells /ml).

From the dilution plate, a 5µL replica is stamped onto a black shallow-well u-bottom 384-well plate (Corning # 3676). To this, 5µL cell suspension is added and the plates incubated at room temperature for 30 minutes.

cAMP levels are measured using a commercially available cAMP dynamic 2 HTRF kit (Cisbio, Cat # 62AM4PEJ), following the two step protocol as per manufacturer's recommendations. In brief; anti-cAMP cryptate (donor fluorophore) and cAMP-d2 (acceptor fluorophore) are made up separately by diluting each 1/20 in conjugate & lysis buffer provided in the kit. 5µL anti-cAMP cryptate is added to all wells of the assay plate, and 5µL cAMP-d2 is added to all wells except non-specific binding (NSB) wells, to which conjugate and lysis buffer are added. Plates are incubated at room temperature for one hour and then read on an Envision (Perkin Elmer) using excitation wavelength of 320nm and emission wavelengths of 620nm & 665nm. EC₅₀ values of the synthetic peptides determined in cAMP assays are then determined.

In additional experiments for determining biological activity/receptor potency, CHO cells with stable recombinant expression of the human, mouse or rat GlucR or GLP-1 receptor are cultured in DMEM 10% FBS and geneticin (100 µg/ml). Cryopreserved cells stocks are prepared in 1x cell freezing medium-DMSO serum free (Sigma Aldrich) at 2x10⁷/vial and stored at -80°C. Cells are rapidly thawed at 37°C and then diluted into assay buffer (DMEM) containing serum albumin at 4.4, 3.2 and 3.2% for human, rat, and mouse serum albumin respectively. Peptides are serially diluted in DMSO and then diluted 100 fold into DMEM containing serum albumin at stated final concentration. Diluted peptides are then transferred into 384 black shallow well microtitre assay plates. Cells are added to the assay plates and incubated for 30 min at room temperature. Following incubation the assay is stopped and cAMP levels measured using the HTRF® dynamic d2 cAMP assay kit available from CisBio Bioassays, as per the manufacturers guidelines. Plates are read on Perkin Elmer ENVISION® fluorescence plate readers. Human and rat serum albumin are purchased from Sigma Aldrich and mouse serum albumin from Equitech Bio Ltd.

Data is transformed to % Delta F as described in the manufacturer's guidelines and analyzed by 4-parameter logistic fit to determine EC₅₀ values. EC₅₀ values determined are dependent on both the intrinsic potency of the peptides tested at the GLP-1 and glucagon receptors in the recombinant cell lines and on the affinity of the peptide for serum albumin, which determines the amount of free peptide. Association with serum albumin increases the EC₅₀ value obtained. The fraction of free peptide at plasma concentrations of albumin and the EC₅₀ at 0% HSA can be calculated based on the variation in cAMP generation with the HSA concentration. To compare the balance of activities at the GLP-1R and GlucR between different peptides and across different conditions, these can be correlated, where the EC₅₀'s are related to those of comparator peptides.

### 4.1.3 Results

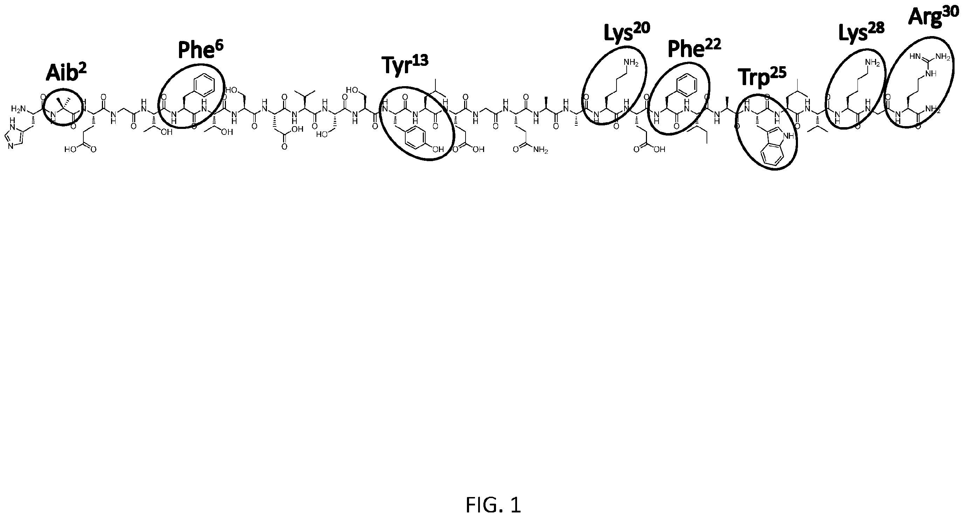

Analysis of enzymatic cleavage of glucagon-like peptide 1 indicated suitable sites for substitution as shown in FIG. 1 to be Aib², Phe⁶, Tyr¹³, Lys²⁰, Phe²², Trp²⁵, Lys²⁸, and Arg³⁰. Shown below in Table 2 is an exemplary design flow showing iterations for developing a synthetic, glucagon-like peptide 1 (GLP-1) as described herein, where these amino acid sites, as well as others, were substituted. It should be recognized that such a design flow can be readily applied to any desired peptide to produce a protease protected peptide as desired.

**TABLE 2**

| **Discussion / Description** | **GLP-1 Peptides incorporating alpha-methyl amino acids** | **SEQ ID NO:** | **Primary assay EC₅₀ (n = 2)** | | |
|---|---|---|---|---|---|
| | | | ***h*Gluc-R** | ***h*GLP-1R** | ***h*GIP-R** |
| Wild-type GLP-1 (7-36) amide | HAEGT⁵ FTSDV¹⁰ SSYLE¹⁵ GQAAK²⁰ EFIAW²⁵ LVKGR³⁰ | **1** | 9.10E-08 | 3.45E-11 | 9.10E-08 |
| Standard GLP-1 comparator against which stability/potency of modified analogues is compared | H-(Aib)²-EGT⁵ FTSDV¹⁰ SSYLE¹⁵ GQAAK²⁰ EFIAW²⁵ LVKGR³⁰ | **4** | 1.02E-07 | 1.365E-11 | 1.02E-07 |
| C-term lipidated GLP-1 comparator, lipid has no apparent effect on potency/selectivity | H-(Aib)²-EGT⁵ FTSDV¹⁰ SSYLE¹⁵ GQAAK²⁰ EFIAW²⁵ LVKGR³⁰-K(ε-Palm) | **5** | 9.06E-08 | 2.54E-11 | 9.06E-08 |
| Replacement of Neprilysin/Chymotrypsin susceptible native Phe⁶ with resistant α-MePhe⁶ | H-(Aib)²-EGT⁵-(α-MeF)⁶-TSDV¹⁰ SSYLE¹⁵ GQAAK²⁰ EFIAW²⁵ LVKGR³⁰ | **6** | 1.53E-07 | 2.6E-11 | 7.49E-08 |
| Comparitor demonstrating that Aib⁶ fullfils does NOT fulfill aromatic requirements of Phe⁶ as well as α-MePhe⁶ | H-(Aib)²-EGT⁵-(Aib)⁶-TSDV¹⁰ SSYLE¹⁵ GQAAK²⁰ EFIAW²⁵ LVKGR³⁰ | **7** | 1.51E-07 | 7.285E-11 | 1.51E-07 |
| Replacement of Neprilysin/Chymotrypsin susceptible native Tyr¹³ with resistant α-MeTvr¹³ | H-(Aib)²-EGT⁵ FTSDV¹⁰ SS-(α-MeY)¹³-LE¹⁵ GQAAK²⁰ EFIAW²⁵ LVKGR³⁰ | **8** | 1.56E-07 | 2.285E-11 | 1.56E-07 |
| Replacement of Neprilysin/Chymotrypsin susceptible Tyr¹³ with α-MePhe¹³ without loss of potency/selectivity | H-(Aib)²-EGT⁵ FTSDV¹⁰ SS-(α-MeF)¹³-LE¹⁵ GQAAK²⁰ EFIAW²⁵ LVKGR³⁰ | **9** | 1.41E-07 | 3.38E-11 | 1.41E-07 |
| Comparitor demonstrating that Aib¹³ does NOT fulfill aromatic requirements of Tyr¹³ as well as α-MeTyr¹³ or α-MePhe¹³ | H-(Aib)²-EGT⁵ FTSDV¹⁰ SS-(Aib)¹³-LE¹⁵ GQAAK²⁰ EFIAW²⁵ LVKGR³⁰ | **10** | 1.31E-07 | 4.375E-11 | 1.31E-07 |
| Replacement of Neprilysin/Chymotrypsin susceptible native Phe²²with resistant α-MePhe²² | H-(Aib)²-EGT⁵ FTSDV¹⁰ SSYLE¹⁵ GQAAK²⁰ E-(α-MeF)²²IAW²⁵ LVKGR³⁰ | **11** | 1.53E-07 | 5.47E-11 | 1.53E-07 |
| Comparitor demonstrating that Aib²² does not fullfil aromatic requirement of Phe²² as well as α-MePhe²² | H-(Aib)²-EGT⁵ FTSDV¹⁰ SSYLE¹⁵ GQAAK²⁰ E-(Aib)²²IAW²⁵ LVKGR³⁰ | **12** | 1.47E-07 | 2.585E-09 | 1.47E-07 |
| Replacement of Neprilysin/Chymotrypsin susceptible native Trp²⁵ with resistant α-MeTrp²⁵ | H-(Aib)²-EGT⁵ FTSDV¹⁰ SSYLE¹⁵ GQAAK²⁰ EFIA-(α-MeW)²⁵ LVKGR³⁰ | **13** | 1.64E-07 | 2.6E-11 | 1.64E-07 |
| Replacement of susceptible Trp²⁵ with more cost effective α-MePhe²⁵ without loss of potency/selectivity | H-(Aib)²-EGT⁵ FTSDV¹⁰ SSYLE¹⁵ GQAAK²⁰ EFIA-(α-MeF)²⁵ LVKGR³⁰ | **14** | 1.70E-07 | 1.96E-11 | 1.70E-07 |
| Comparitor demonstrating that Aib²⁵ does not fullfil aromatic requirement of Trp²⁵ as well as α-MeTrp²⁵ or α-MePhe²⁵ | H-(Aib)²-EGT⁵ FTSDV¹⁰ SSYLE¹⁵ GQAAK²⁰ EFIA-(Aib)²⁵ LVKGR³⁰ | **15** | 1.77E-07 | 4.65E-11 | 1.77E-07 |
| Replacing all aromatics with Aib results in complete loss of potency/selectivity | H-(Aib)²-EGT⁵-(Aib)⁶-TSDV¹⁰SS-(Aib)¹³-LE¹⁵ GQAAK²⁰E-(Aib)²²-IA-(Aib)²⁵ LVKGR³⁰ | **16** | 1.02E-07 | 1.02E-07 | 1.02E-07 |
| Replacing all aromatics with Norleucine restores some potency/selectivity | H-(Aib)²-EGT⁵-(Nle)⁶-TSDV¹⁰ SS-(Nle)¹³-LE¹⁵ GQAAK²⁰ E-(Nle)²²-IA-(Nle)²⁵ LVKGR³⁰ | **17** | 1.02E-07 | 3.475E-09 | 1.02E-07 |
| Replacing Tyr¹³ and Trp²⁵ with Phe is fully tolerated with no loss of potency or selectivity | H-(Aib)²-EGT⁵-FTSDV¹⁰ SS-(F)¹³-LE¹⁵ GQAAK²⁰ EFIA-(F)²⁵ LVKGR³⁰ | **18** | 1.02E-07 | 1.625E-11 | 1.02E-07 |
| Replacing all aromatics with Neprilysin/Chymotrypsin resistant α-MePhe fully tolerated with no loss of potency/selectivity | H-(Aib)²-EGT⁵-(α-MeF)⁶-TSDV¹⁰ SS-(α-MeF)¹³-LE¹⁵ GQAAK²⁰ E-(α-MeF)²²-IA-(α-MeF)²⁵ LVKGR³⁰ | **19** | 1.02E-07 | 2.6E-11 | 1.02E-07 |
| Replacing Trypsin/Kallikrein susceptible Lys²⁰ with α-MeLys²⁰ maintains full potency/selectivity profile | H-(Aib)²-EGT⁵ FTSDV¹⁰ SSYLE¹⁵ GQAA-(α-MeK)²⁰ EFIAW²⁵ LVKGR³⁰ | **20** | 1.10E-07 | 1.67E-11 | 1.10E-07 |
| Comparitor demonstrating that Aib²⁰ almost fulfills basic requirement of Lys²⁰ as well as α-MeLys²⁰ | H-(Aib)²-EGT⁵ FTSDV¹⁰ SSYLE¹⁵ GQAA-(Aib)²⁰ EFIAW²⁵ LVKGR³⁰ | **21** | 7.65E-08 | 1.995E-11 | 7.65E-08 |
| Replacing Trypsin/Kallikrein susceptible Lys²⁸ with α-MeLys²⁸ maintains full potency/selectivity profile | H-(Aib)²-EGT⁵ FTSDV¹⁰ SSYLE¹⁵ GQAAK²⁰ EFIAW²⁵ LV-(α-MeK)²⁸-GR³⁰ | **22** | 9.14E-08 | 1.9E-11 | 9.14E-08 |
| Comparitor demonstrating that Aib²⁸ almost fulfills basic requirement of Lys²⁸ as well as α-MeLys²⁸ | H-(Aib)²-EGT⁵ FTSDV¹⁰ SSYLE¹⁵ GQAAK²⁰ EFIAW²⁵LV-(Aib)²⁸-GR³⁰ | **23** | 1.03E-07 | 2.79E-11 | 1.05E-07 |
| Replacing Trypsin/Kallikrein susceptible Arg³⁰ with α-MeArg³⁰ maintains full potency/selectivity profile | H-(Aib)²-EGT⁵ FTSDV¹⁰ SSYLE¹⁵ GQAAK²⁰ EFIAW²⁵ LVKG-(α-MeR)³⁰ | **24** | 1.11E-07 | 2.105E-11 | 1.11E-07 |
| Comparitor demonstrating that Aib³⁰ almost fullfils basic requirement of Arg³⁰ as well as α-MeArg³⁰ | H-(Aib)²-EGT⁵ FTSDV¹⁰ SSYLE¹⁵ GQAAK²⁰ EFIAW²⁵ LVKG-(Aib)³⁰ | **25** | 9.64E-08 | 2.765E-11 | 9.64E-08 |
| Replacing basic residues with Trypsin-resistant α-Methyl residues maintains full potency/selectivity profile | H-(Aib)²-EGT⁵ FTSDV¹⁰ SSYLE¹⁵GQAA-(α-MeK)²⁰ EFIAW²⁵ LV-(α-MeK)²⁸-G-(α-MeR)³⁰ | **26** | 1.09E-07 | 1.895E-11 | 1.09E-07 |
| Comparitor demonstrating Aib in positions 20,28 and 30 also fullfils basic requirements of GLP-1 | H-(Aib)²-EGT⁵ FTSDV¹⁰ SSYLE¹⁵ GQAA-(Aib)²⁰ EFIAW²⁵ LV-(Aib)²⁸-G-(Aib)³⁰ | **27** | 1.45E-07 | 2.065E-11 | 1.45E-07 |
| Replacing bulkyThr⁵ with Ser⁵ results in more efficient coupling following α-MePhe⁶ without loss of potency/selectivity | H-(Aib)²-EG-(S)⁵-FTSDV¹⁰ SSYLE¹⁵ GQAAK²⁰ EFIAW²⁵ LVKGR³⁰ | **28** | 1.02E-07 | 9.51E-12 | 1.02E-07 |
| α-MePhe in positions 6,13 and 22 maintains native potency/selectivity (direct comparator to Ser⁵ analogue) | H-(Aib)²-EGT⁵-(α-MeF)⁶-TSDV¹⁰ SS-(α-MeF)¹³-LE¹⁵ GQAAK²⁰ E-(α-MeF)²²-IAW²⁵ LVKGR³⁰ | **29** | 1.02E-07 | 1.255E-11 | 1.02E-07 |
| α-MePhe in positions 6,13 and 25 maintains native potency/selectivity (direct comparator to Ser⁵ analogue) | H-(Aib)²-EGT⁵-(α-MeF)⁶-TSDV¹⁰ SS-(α-MeF)¹³-LE¹⁵ GQAAK²⁰ EF²²-IA-(α-MeF)²⁵ LVKGR³⁰ | **30** | 1.02E-07 | 1.165E-11 | 9.89E-08 |
| α-MePhe in positions 6, 22 and 25 maintains native potency/selectivity (direct comparator to Ser⁵ analogue) | H-(Aib)²-EGT⁵-(α-MeF)⁶-TSDV¹⁰ SSYLE¹⁵ GQAAK²⁰ E-(α-MeF)²²-IA-(α-MeF)²⁵ LVKGR³⁰ | **31** | 1.02E-07 | 1.555E-11 | 1.02E-07 |
| α-MePhe in positions 13, 22 and 25 maintains native potency/selectivity | H-(Aib)²-EGT⁵-FTSDV¹⁰ SS-(α-MeF)¹³-LE¹⁵ GQAAK²⁰ E-(α-MeF)²²-IA-(α-MeF)²⁵ LVKGR³⁰ | **32** | 1.02E-07 | 1.019E-10 | 1.02E-07 |
| Ser⁵ incorporation, Tyr¹³ and Trp²⁵ replaced with Phe. No loss of potency/selectivity | H-(Aib)²-EG-(S)⁵-FTSDV¹⁰ SS-(F)¹³-LE¹⁵ GQAAK²⁰ EFIA-(F)²⁵ LVKGR³⁰ | **33** | 1.02E-07 | 1.825E-11 | 1.02E-07 |
| α-MePhe in positions 6,13 and 22 maintains native potency/selectivity (direct comparator to Thr⁵ analogue) | H-(Aib)²-EG-(S)⁵-(α-MeF)⁶-TSDV¹⁰ SS-(α-MeF)¹³-LE¹⁵ GQAAK²⁰ E-(α-MeF)²²-IAW²⁵ LVKGR³⁰ | **34** | 1.02E-07 | 2.25E-11 | 1.02E-07 |
| α-MePhe in positions 6,13 and 25 maintains native potency/selectivity (direct comparator to Thr⁵ analogue) | H-(Aib )²-EG-(S)⁵-(α-MeF)⁶-TSDV¹⁰ SS-(α-MeF)¹³-LE¹⁵ GQAAK²⁰ EFIA-(α-MeF)²⁵LVKGR³⁰ | **35** | 1.02E-07 | 2.01E-11 | 1.02E-07 |
| α-MePhe in positions 6, 22 and 25 maintains native potency/selectivity (direct comparator to Thr⁵ analogue) | H-(Aib)²-EG-(S)⁵-(α-MeF)⁶-TSDV¹⁰ SSYLE¹⁵ GQAAK²⁰ E-(α-MeF)²²-IA-(α-MeF)²⁵ LVKGR³⁰ | **36** | 1.02E-07 | 2.665E-11 | 1.02E-07 |
| α-MePhe in positions 13, 22 and 25 maintains native potency/selectivity (direct comparator to Thr⁵ analogue) | H-(Aib)²-EG-(S)⁵-FTSDV¹⁰ SS-(α-MeF)¹³-LE¹⁵ GQAAK²⁰ E-(α-MeF)²²-IA-(α-MeF)²⁵ LVKGR³⁰ | **37** | 1.02E-07 | 1.91E-10 | 1.02E-07 |
| Ser⁵ + aromatics replaced with α-MePhe results in improved synthesis yield, fully Neprilysin resistant but Trypsin susceptibility | H-(Aib)²-EG-(S)⁵-(α-MeF)⁶-TSDV¹⁰SS-(α-MeF)¹³-LE¹⁵ GQAAK²⁰ E-(α-MeF)²²-IA-(α-MeF)²⁵ LVKGR³⁰ | **38** | 1.02E-07 | 3.385E-11 | 1.02E-07 |
| Replacing Arg³⁰ with non-Trypsin susceptible Gly³⁰ fully tolerated with no loss potency or selectivity, cheaper than α-MeArg³⁰ | H-(Aib)²-EG-(S)⁵-(α-MeF)⁶-TSDV¹⁰ SS-(α-MeF)¹³-LE¹⁵GQAAK²⁰ E-(α-MeF)²²-IA-(α-MeF)²⁵ LVKG-(G)³⁰ | **39** | 1.02E-07 | 3.225E-11 | 1.02E-07 |
| Replacing Chymotrypsin susceptible Val²⁷ with Aib²⁷ overcomes cleavage but results in some some lost potency and has poor solubility | H-(Aib)²-EG-(S)⁵-(α-MeF)⁶-TSDV¹⁰ SS-(α-MeF)¹³-LE¹⁵ GQAAK²⁰ E-(α-MeF)²²-IA-(α-MeF)²⁵ L-(Aib)²⁷-KGR³⁰ | **40** | 1.02E-07 | 2.54E-10 | 1.02E-07 |
| Replacing Chymotrypsin susceptible Val²⁷ with α-MeVal²⁷ overcomes cleavage restores potency but has poor solubility | H-(Aib)²-EG-(S)⁵-(α-MeF)⁶-TSDV¹⁰ SS-(α-MeF)¹³-LE¹⁵ GQAAK²⁰ E-(α-MeF)²²-IA-(α-MeF)²⁵ L-(α-MeV)²⁷-KGR³⁰ | **41** | 1.02E-07 | 5.455E-11 | 1.02E-07 |
| Aib²⁹ offers some protection to both Lys²⁸ and Arg³⁰ showing dual protection effect of α-Methyl residues in general | H-(Aib)²-EG-(S)⁵-(α-MeF)⁶-TSDV¹⁰ SS-(α-MeF)¹³-LE¹⁵GQAAK²⁰ E-(α-MeF)²²-IA-(α-MeF)²⁵ LVK-(Aib)²⁹-R³⁰ | **42** | 1.02E-07 | 2.335E-11 | 1.02E-07 |
| Aib in positions 27 and 29 remove Val²⁷ liability and protect both Lys²⁸ and Arg³⁰ against Trypsin however solubility is poor | H-(Aib)²-EG-(S)⁵-(α-MeF)⁶-TSDV¹⁰SS-(α-MeF)¹³-LE¹⁵ GQAAK²⁰ E-(α-MeF)²²-IA-(α-MeF)²⁵ L-(Aib)²⁷-K-(Aib)²⁹-R³⁰ | **43** | 1.02E-07 | 7.07E-11 | 1.02E-07 |
| Incorporating Gly³⁰ alongside α-MeLys²⁰ + all legacy modifications restores solubility, maintains potency/selectivity | H-(Aib )²-EG-(S)⁵-(α-MeF)⁶-TSDV¹⁰ SS-(α-MeF)¹³-LE¹⁵ GQAA-(α-MeK)²⁰E-(α-MeF)²²-IA-(α-MeF)²⁵ LVKG-(G)³⁰ | **44** | 1.04E-07 | 9.855E-12 | 1.04E-07 |
| Incorporating Gly³⁰ alongside α-MeLys²⁸ + all legacy modifications restores solubility, maintains potency/selectivity | H-(Aib)²-EG-(S)⁵-(α-MeF)⁶-TSDV¹⁰ SS-(α-MeF)¹³-LE¹⁵ GQAAK²⁰ E-(α-MeF)²²-IA-(α-MeF)²⁵ LV-(α-MeK)²⁸-G-(G)³⁰ | **45** | 1.04E-07 | 2.23E-11 | 1.04E-07 |
| Gly³⁰ + α-MeLys in positions 20 and 28 results in Neprilysin/Trypsin resistance, maintaining solubility/ potency/selectivity | H-(Aib)²-EG-(S)⁵-(α-MeF)⁶-TSDV¹⁰ SS-(α-MeF)¹³-LE¹⁵ GQAA-(α-MeK)²⁰E-(α-MeF)²²-IA-(α-MeF)²⁵ LV-(α-MeK)²¹-G-(G)³⁰ | **46** | 1.03E-07 | 1.405E-11 | 1.03E-07 |
| C-terminal lipidation maintains solubility, potency and selectivity. Allows some rat serum studies to be conducted | H-(Aib)²-EG-(S)⁵-(α-MeF)⁶-TSDV¹⁰ SS-(α-MeF)¹³-LE¹⁵ GQAA-(α-MeK)²⁰E-(α-MeF)²²-IA-(α-MeF)²⁵ LV-(α-MeK)²⁸-G-(G)³⁰-K(ε-Palm) | **47** | 9.27E-08 | 1.05 1E-10 | 9.27E-08 |
| Addition of flexible linker (SSG)₃ for potential ADC-conjugation approach | H-(Aib)²-EG-(S)⁵-(α-MeF)⁶-TSDV¹⁰ SS-(α-MeF)¹³-LE¹⁵ GQAAK²⁰ E-(α-MeF)²²-IA-(α-MeF)²⁵ LVKG-(G)³⁰-(SSG)₃-K | **48** | 8.29E-08 | 2.38E-11 | 8.29E-08 |
| Addition of recombinant style flexible linker (SSG)₃ with Lys(γ-Glu)-Palm lipid (albumin tag) for extended circulatory half-life | H-(Aib)²-EG-(S)⁵-(α-MeF)⁶-TSDV¹⁰ SS-(α-MeF)¹³-LE¹⁵ GQAAK²⁰ E-(α-MeF)²²-IA-(α-MeF)²⁵ LVKG-(G³⁰-(SSG)₃-K(ε-γ-E-Palm) | **49** | 7.59E-08 | 9.29E-12 | 4.09E-08 |
| Addition of recombinant style flexible linker (SSG)₃ with 40kD mPEG for extended circulatory half life | H-(Aib)²-EG-(S)⁵-(α-MeF)⁶-TSDV¹⁰ SS-(α-MeF)¹³-LE¹⁵ GQAAK²⁰ E-(α-MeF)²²-IA-(α-MeF)²⁵ L VKG-(G)³⁰-(SSG)₃-(Cys-Mal-mPEG) [40kD] | **50** | 4.13E-09 | 2.78E-11 | 4.13E-09 |
| Start of Val²⁶ series, overcoming Chymotrypsin liability, maintaining solubility/ potency/selectivity minimizing unnatural residues | H-(Aib)²-EG-(S)⁵-(α-MeF)⁶-TSDV¹⁰ SS-(α-MeF)¹³-LE¹⁵ GQAA-(α-MeK)²⁰E-(α-MeF)²²-IA-(α-MeF)²⁵-(V)²⁶-V-(α-MeK)²⁸-G-(G)³⁰ | **51** | 1.04E-07 | 2.19E-11 | 1.04E-07 |
| Lipidated for PK studies, excellent enzyme resistance (DPP-IV, Neprilysin, Chymotrypsin, Trypsin, Pepsin) good potency/selectivity/solubility | H-(Aib)²-EG-(S)⁵-(α-MeF)⁶-TSDV¹⁰ SS-(α-MeF)¹³-LE¹⁵ GQAA-(α-MeK)²⁰E-(α-MeF)²²-IA-(α-MeF)²⁵-(V)²⁶-V-(α-MeK)²⁸-G-(G)³⁰-K(ε-Palm) | **52** | 9.31E-08 | 4.1E-10 | 9.31E-08 |
| Tetrazolyl lapidated for maintaining enzyme resistance (DPP-IV, Neprilysin, Chymotrypsin, Trypsin, Pepsin) improvimg solubility/potency adding some GIP/GLUC triple agonism Excellent enzyme resistance (DPP-IV, Neprilysin, Chymotrypsin, Trypsin, Pepsin) good potency/selectivity | H-(Aib)²-EG-(S)⁵-(α-MeF)⁶-TSDV¹⁰ SS-(α-MeF)¹³-LE¹⁵ GQAA-(α-MeK)²⁰E-(α-MeF)²²-IA-(α-MeF)²⁵-(V)²⁶-V-(α-MeK)²⁸-G-(G)³⁰-K(ε-Tetrazolylpalm) | **53** | 9.78E-09 | 4.51E-11 | 9.78E-09 |
| Effect of linker on solubility/potency/selectivity Excellent enzyme resistance (DPP-IV, Neprilysin, Chymotrypsin, Trypsin, Pepsin) good potency/selectivity | H-(Aib)²-EG-(S)⁵-(α-MeF)⁶-TSDV¹⁰ SS-(α-MeF)¹³-LE¹⁵ GQAA-(α-MeK)²⁰E-(α-MeF)²²-IA-(α-MeF)²⁵-(V)²⁶-V-(α-MeK)²⁸-G-(G)³⁰-K(ε-γ-E-Palm) | **54** | Not tested | Not tested | Not tested |
| Effect of linker on solubility/potency/selectivity Excellent enzyme resistance (DPP-IV, Neprilysin, Chymotrypsin, Trypsin, Pepsin) good potency/selectivity | H-(Aib)²-EG-(S)⁵-(α-MeF)⁶-TSDV¹⁰ SS-(α-MeF)¹³-LE¹⁵ GQAA-(α-MeK)²⁰E-(α-MeF)²²-IA-(α-MeF)²⁵-(V)²⁶-V-(α-MeK)²⁸-G-(G)³⁰-K(ε-γ-E-Tetrazolylpalm) | **55** | Not tested | Not tested | Not tested |
| Effect of linker on solubility/potency/selectivity Excellent enzyme resistance (DPP-IV, Neprilysin, Chymotrypsin, Trypsin, Pepsin) good potency/selectivity | H-(Aib)²-EG-(S)⁵-(α-MeF)⁶-TSDV¹⁰ SS-(α-MeF)¹³-LE¹⁵ GQAA-(α-MeK)²⁰E-(α-MeF)²²-IA-(α-MeF)²⁵-(V)²⁶-V-(α-MeK)²⁸-G-(G)³⁰-K(ε-γ-E-(PEG)₂-Tetrazolylpalm) | **56** | Not tested | Not tested | Not tested |
| Assessing potency of GLP-1 with α-Methyl residues GLP-1 incorporating α-Methyl residues bearing native sidechains in multiple peptidase-liable positions | H-(Aib)²-EG-(S)⁵-(α-MeF)⁶-TSDV¹⁰ SS-(α-MeY)¹³-LE¹⁵ GQAA-(α-MeK)²⁰E-(α-MeF)²²-IA-(α-MeW)²⁵ LV-(α-MeK)²⁸-G-(α-MeR)³⁰ | **57** | 1.99E-08 | 2.71E-11 | 1.39E-07 |
| Lipidated GLP-1 α-Methyl residues bearing native sidechains in multiple peptidase-liable positions | H-(Aib)²-EG-(S)⁵-(α-MeF)⁶-TSDV¹⁰ SS-(α-MeY)¹³-LE¹⁵ GQAA-(α-MeK)²⁰E-(α-MeF)²²-IA-(α-MeW)²⁵ LV-(α-MeK)²⁸-G-(α-MeR)³⁰-K(ε-γ-E-Palm) | **58** | 1.05E-07 | 8.485E-11 | 1.05E-07 |
| Excellent enzyme resistance (DPP-IV, Neprilysin, Chymotrypsin, Trypsin, Pepsin) good potency/selectivity | H-(Aib)²-EG-(S)⁵-(α-MeF)⁶-TSDV¹⁰SS-(α-MeF)¹³-LE¹⁵G-(E)¹⁷-AA-(α-MeK)²⁰E-(α-MeF)²²-IA-(α-MeF)²⁵-(V)²⁶-V-(α-MeK)²⁸-G-(G)³⁰-K(ε-γ-E-Palm) | **59** | 8.98E-08 | 2.175E-10 | 8.98E-08 |
| Excellent enzyme resistance (DPP-IV, Neprilysin, Chymotrypsin, Trypsin, Pepsin) good potency/selectivity | H-(Aib)²-EG-(S)⁵-(α-MeF)⁶-TSDV¹⁰SS-(α-MeF)¹³-LE¹⁵G-(E)¹⁷-AA-(α-MeK)²⁰E-(α-MeF)²²-IA-(α-MeF)²⁵-(V)²⁶-V-(α-MeK)²⁸-G-(G)³⁰ | **60** | 6.63E-08 | 2.12E-11 | 1.04E-07 |
| Excellent enzyme resistance (DPP-IV, Neprilysin, Chymotrypsin, Trypsin, Pepsin) good potency/selectivity | H-(Aib)²-EG-(S)⁵-(α-MeF)⁶-TSDV¹⁰SS-(α-MeF)¹³-LE¹⁵G-(E)¹⁷-AA-(α-MeK)²⁰E-(α-MeF)²²-IA-(α-MeF)²⁵-(V)²⁶-V-(α-MeK)²⁸-G-(G)³⁰-(K)³¹ | **61** | 3.04E-08 | 3.4E-11 | 9.97E-08 |
| Replacing all aromatics with β,β-dimethyl-phenylalanine results in complete loss of potency / selectivity | H-(Aib)²-EGT⁵-(β,β-di-Me-Phe)⁶-TSDV¹⁰SS-(β,β-di-Me-Phe)¹³-LE¹⁵GQAAK²⁰ E-(β,β-di-Me-Phe)²²-IA-(β,β-di-Me-Phe)²⁵ LVKGR³⁰ | **62** | 9.89E-08 | 9.89E-08 | 9.89E-08 |

The stability of these various peptides after exposure to select proteases, as well as EC₅₀ determinations, was then used to guide selection of desired synthetic peptides.

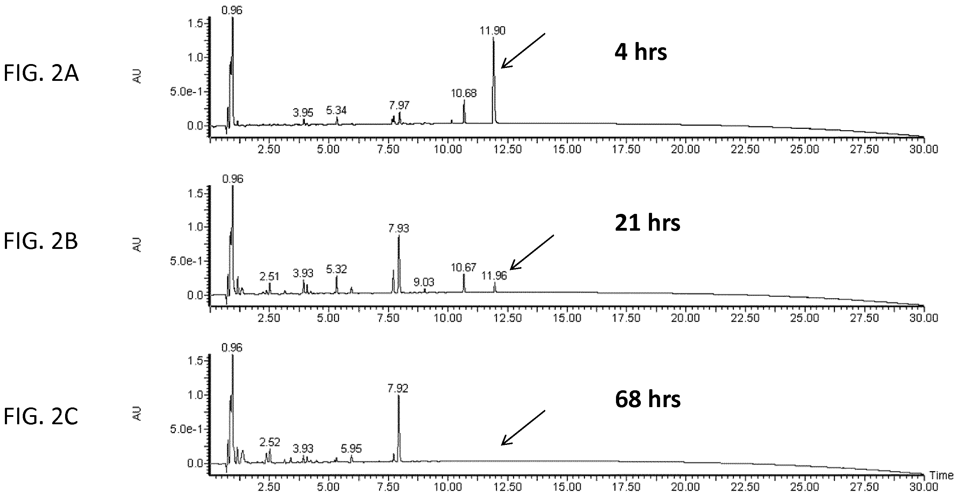

FIGs. 2A-2C show the results of a neprilysin stability study on the standard GLP-1 comparator against which stability/potency of modified analogues was compared, H-(Aib)²-EGT⁵ FTSDV¹⁰ SSYLE¹⁵ GQAAK²⁰ EFIAW²⁵ LVKGR³⁰, SEQ ID NO:4. Arrows show the position of the original peak, and the degradation at 4 hours, 21 hours and 68 hours after incubation with the protease. As shown, rapid degradation occurred at the amino-terminus of all four aromatic residues, with the peptide being completely degraded by 24 hours.

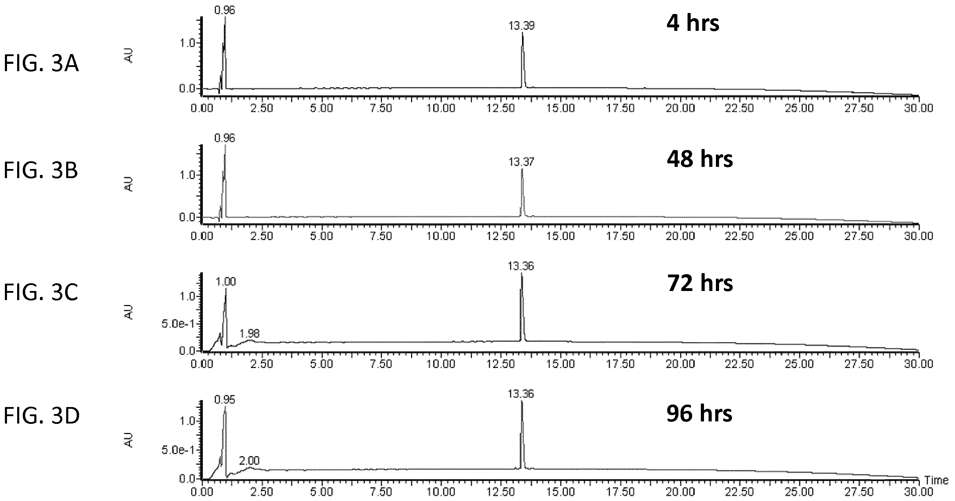

FIGs. 3A-3D show the results of a neprilysin stability study on the synthetic GLP-1 peptide, H-(Aib)²-EG-(S)⁵-(α-MeF)⁶-TSDV¹⁰SS-(α-MeF)¹³-LE¹⁵GQAAK²⁰E-(α-MeF)²²-IA-(α-MeF)²⁵LVKGR³⁰, SEQ ID NO: 38. As demonstrated, the synthetic GLP-1 peptide with alpha-methyl phenylalanine substituted at positions Phe6, Tyr13, Phe22 and Trp25, as well as substitution of serine for threonine at position 5, showed no proteolytic degradation over a 96 hour time-course. Potency measurements made as described herein indicated the synthetic GLP-1 peptide was equipotent to the GLP-1 comparator peptide, SEQ ID NO:4.

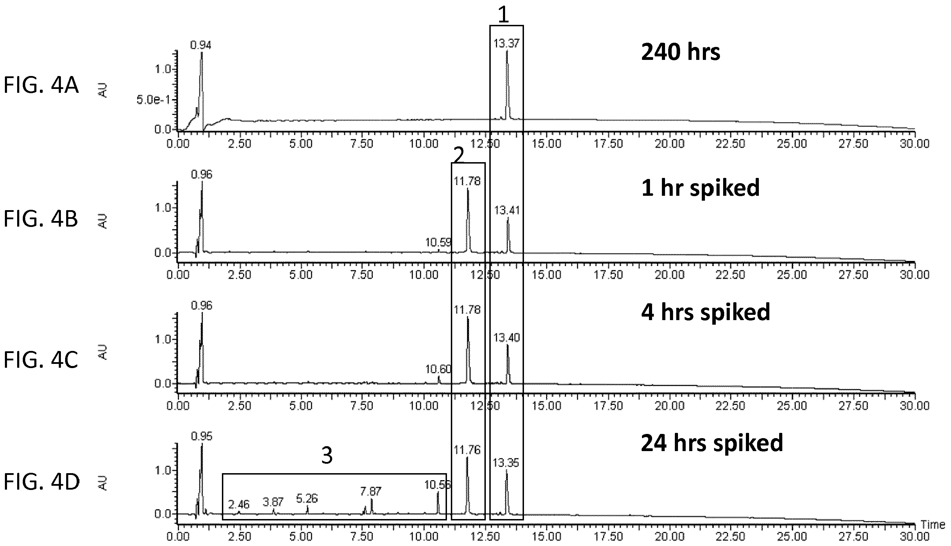

To demonstrate that the neprilysin enzyme was still active in the experiment, GLP-1 comparator peptide, SEQ ID NO:4, was added after 240 hours. As shown in FIG. 4A, the GLP-1 synthetic peptide of SEQ ID NO: 38 was still stable after 10 days. *(See* Box 1 in FIGS. 4A-4D.) In FIGS. 4B-4D, addition of the comparator peptide quickly began to degrade after only 1 hour (*see* Box 2), with significant degradation occurring by 24 hours (*see* Box 3).

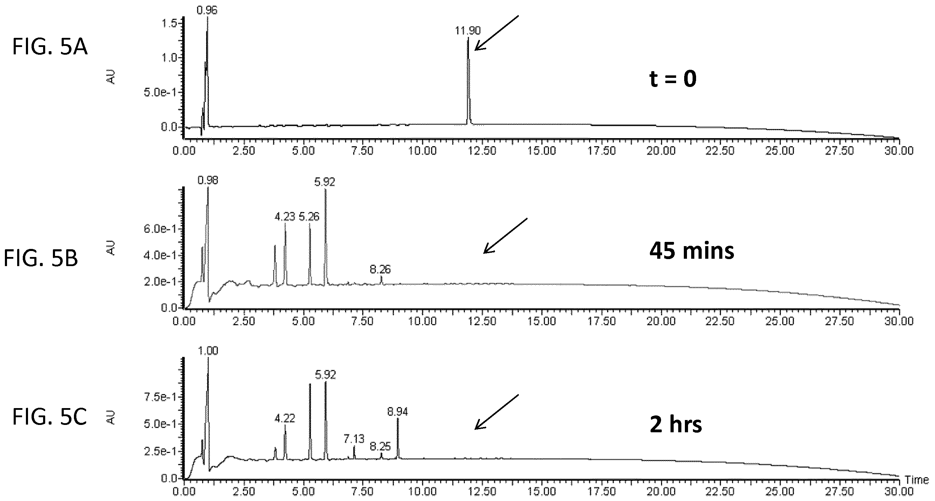

FIGs. 5A-5C show the results of a chymotrypsin stability study on the standard GLP-1 comparator, SEQ ID NO:4. Arrows show the position of the original peak, and the degradation at 45 minutes and 2 hours after incubation with the protease. As shown, rapid degradation occurred at the carboxyl-terminus of all hydrophobic residues, with the peptide being completely degraded by 45 minutes.

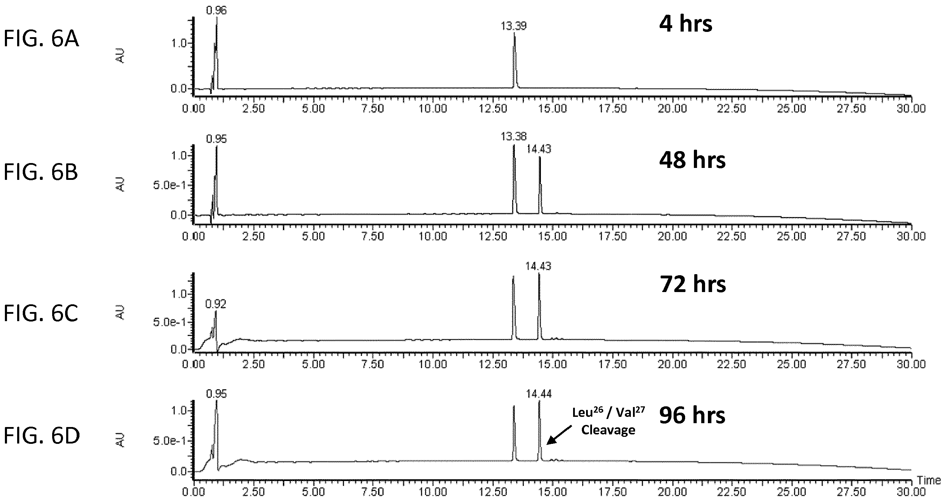

FIGs. 6A-6C show the results of a chymotrypsin stability study on the synthetic GLP-1 peptide, SEQ ID NO: 38. As demonstrated, the synthetic GLP-1 peptide showed degradation occurring by 48 hours, with cleavage observed solely at the Leu26/Val27.

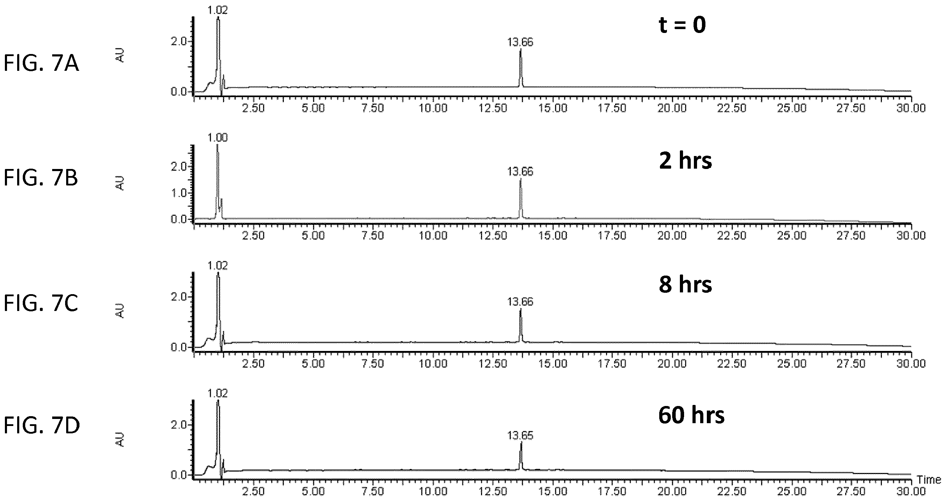

FIGs. 7A-7C show the results of a chymotrypsin stability study on the synthetic GLP-1 peptide, H-(Aib)²-EG-(S)⁵-(α-MeF)⁶-TSDV¹⁰SS-(α-MeF)¹³-LE¹⁵GQAA-(α-MeK)²⁰E-(α-MeF)²²-IA-(α-MeF)²⁵-(V)²⁶-V-(α-MeK)²⁸-G-(G)30, SEQ ID NO: 51. As demonstrated, substitution of leucine 26 to valine resulted in the synthetic GLP-1 peptide demonstrating stability for over 60 hours, with no major cleavage products observed.

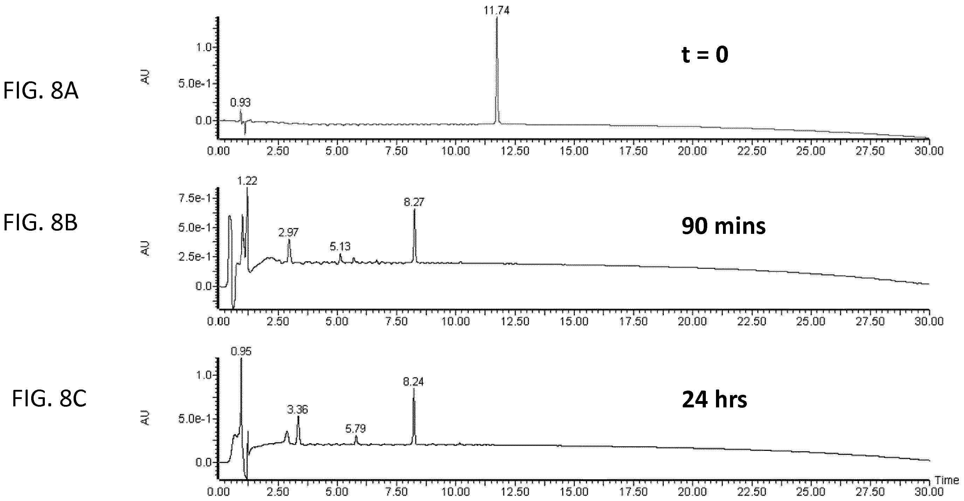

FIGs. 8A-8C show the results of a trypsin stability study on the standard GLP-1 comparator, SEQ ID NO:4. Rapid proteolytic degradation occurred at the carboxyl side of Lys²⁰, Lys²⁸ and Arg³⁰, by 90 minutes.

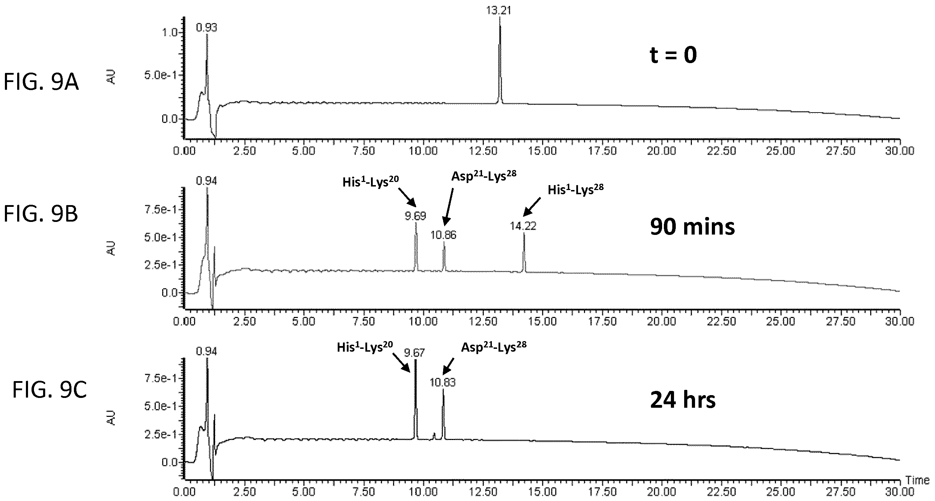

FIGs. 9A-9C show the results of a trypsin stability study on the synthetic GLP-1 peptide, SEQ ID NO: 38. As demonstrated, the synthetic GLP-1 peptide showed degradation occurring by 90 minutes at the carboxyl-side of Lys²⁰, Lys²⁸ and Arg³⁰.

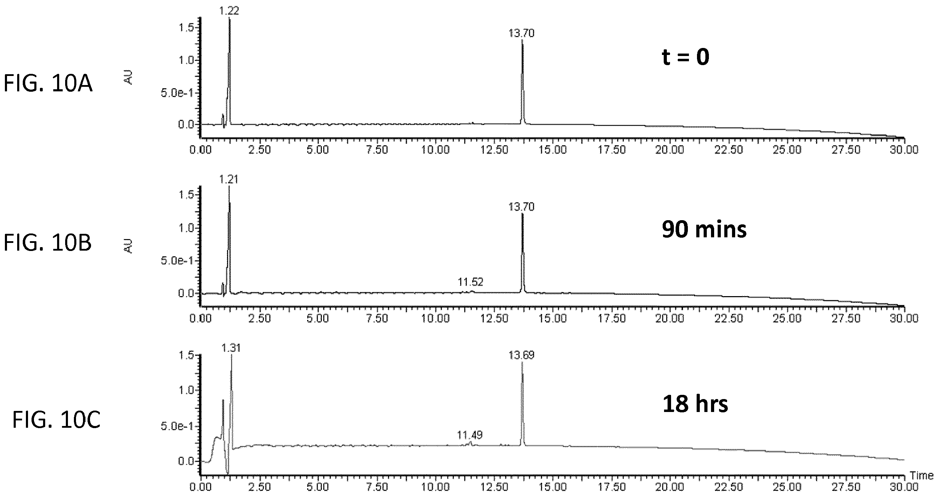

FIGs. 10A-10C show the results of a trypsin stability study on the synthetic GLP-1 peptide, SEQ ID NO: 51. As demonstrated, substitution of both Lys20 and Lys28 by alpha-methyl Lysine, Arg30 by Gly30 and Leu26 by Val26 resulted in the synthetic GLP-1 peptide demonstrating significantly extended stability for over 18 hours.

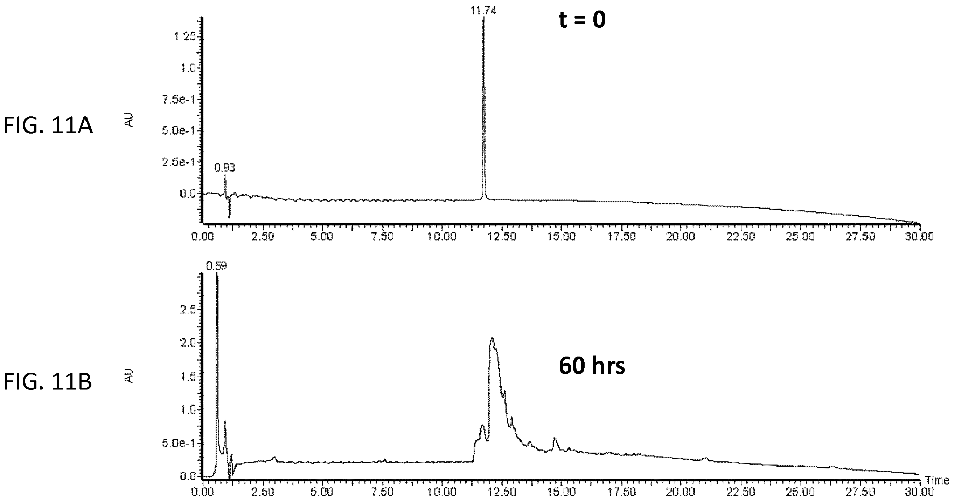

FIGs. 11A-11B show the results of a serum stability study on the standard GLP-1 comparator, SEQ ID NO:4. Rapid proteolytic degradation occurred after 60 hours, resulting in a trace of intact peptide, with significant autolysis of serum proteases creating peptide fragments that occlude the spectrum.

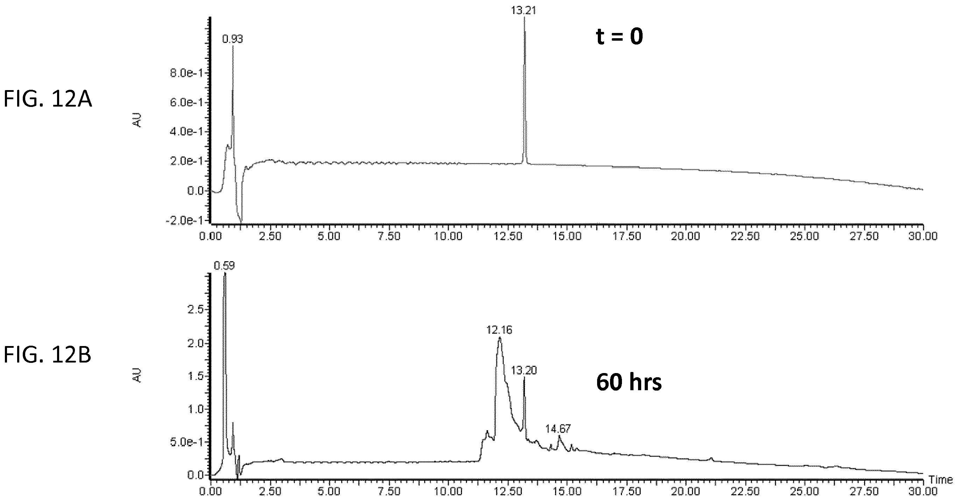

FIGs. 12A-12B show the results of a serum stability study on the synthetic GLP-1 peptide, SEQ ID NO: 38. After 60 hours, approximately 64% of the peptide remains intact, with autolysis of serum proteases creating peptide fragments that occlude the spectrum.

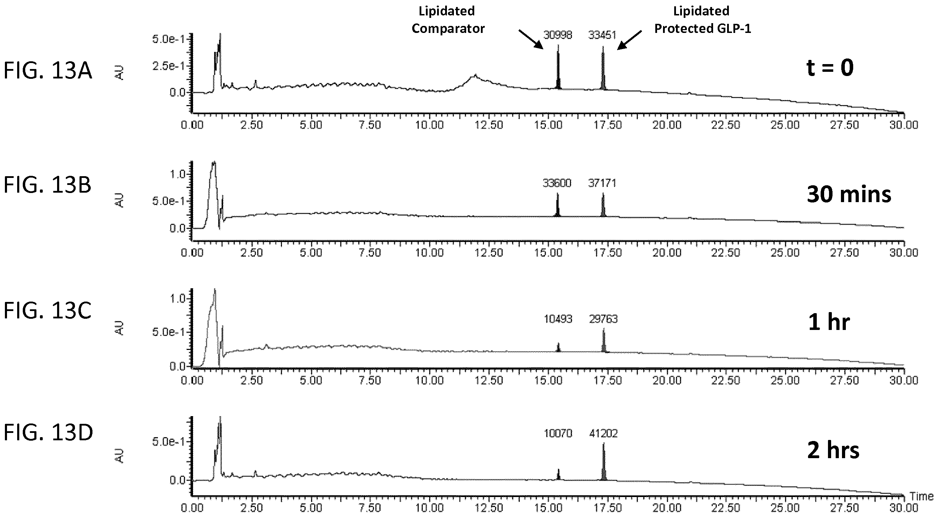

FIGs. 13A-13D show the results of a gastric fluid stability study on a lipidated comparator GLP-1 peptide, H-(Aib)²-EGT⁵FTSDV¹⁰SSYLE¹⁵GQAAK²⁰EFIAW²⁵ LVKGR³⁰-(K-Palm), SEQ ID NO: 5, and a lipidated, protease protected GLP-1 peptide, H-(Aib)²-EG-(S)⁵-(α-MeF)⁶-TSDV¹⁰SS-(α-MeF)¹³-LE¹⁵GQAA-(α-MeK)²⁰E-(α-MeF)²²-IA-(α-MeF)²⁵-(V)²⁶-V-(α-MeK)²⁸-G-(G)³⁰-K(palm), SEQ ID NO: 52. The stability of the lipidated, protease protected GLP-1 protein significantly exceeds that of that lipidated comparator.

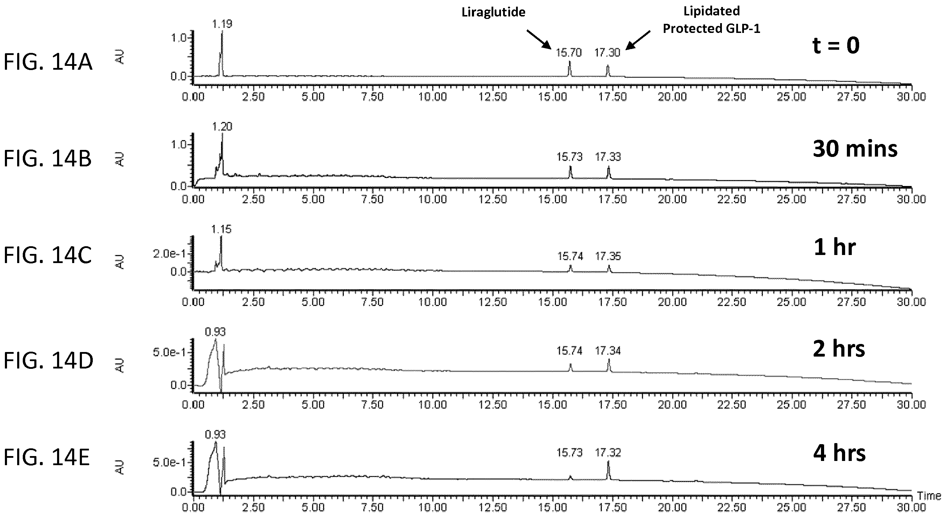

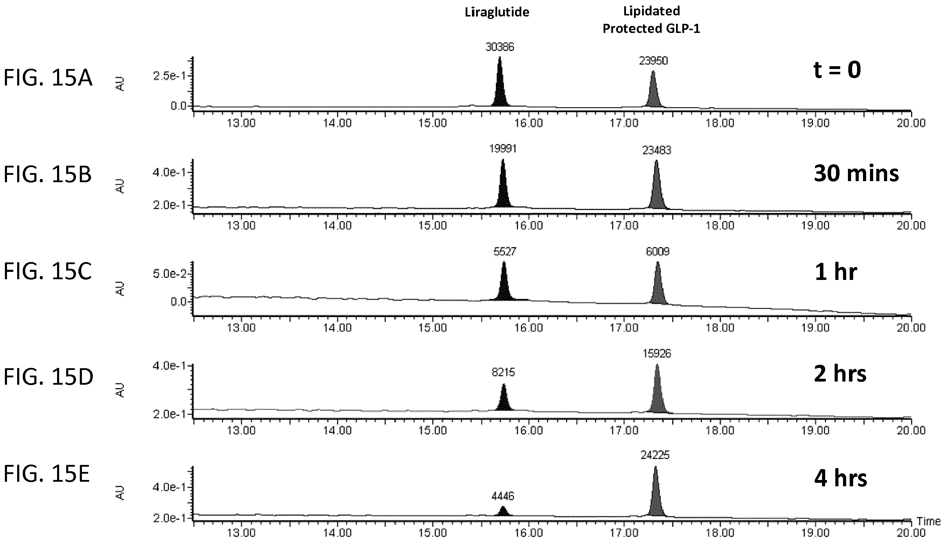

FIGs. 14A-14E show the results of a gastric fluid stability study on a commercially available GLP-1 agonist (Liraglutide, Novo Nordisk) as compared to the lipidated, protease-resistant SEQ ID NO: 52. The stability of the lipidated, protease-resistant GLP-1 peptide significantly exceeds that of Liraglutide. The significant difference in stability is demonstrated even further in FIGs. 15A-15E, showing zoomed spectra, indicating the virtually unchanged spectrum for the protected GLP-1 peptide, SEQ ID NO: 52, over the time course.

All documents, patents, journal articles and other materials cited in the present application are hereby incorporated by reference.

Although the present invention has been fully described in conjunction with several embodiments thereof with reference to the accompanying drawings, it is to be understood that various changes and modifications can be apparent to those skilled in the art. Such changes and modifications are to be understood as included within the scope of the present invention as defined by the appended claims, unless they depart there from.

### EMBODIMENTS:

1. A synthetic peptide comprising at least one substitution of an alpha-methyl functionalized amino acid for a native amino acid residue, wherein the synthetic peptide maintains substantially the same receptor potency and selectivity as a corresponding synthetic peptide that does not comprise the substitutions.
2. The synthetic peptide of embodiment 1, wherein the at least one alpha-methyl functionalized amino acid correspond to the substituted native amino acid residue.
3. The synthetic peptide of embodiment 1, wherein the at least one alpha-methyl functionalized amino acid is selected from the group consisting of alpha-methyl Histidine, alpha-methyl Alanine, alpha-methyl Isoleucine, alpha-methyl Arginine, alpha-methyl Leucine, alpha-methyl Asparagine, alpha-methyl Lysine, alpha-methyl Aspartic acid, alpha-methyl Methionine, alpha-methyl Cysteine, alpha-methyl Phenylalanine, alpha-methyl Glutamic acid, alpha-methyl Threonine, alpha-methyl Glutamine, alpha-methyl Tryptophan, alpha-methyl Glycine, alpha-methyl Valine, alpha-methyl Ornithine, alpha-methyl Proline, alpha-methyl Selenocysteine, alpha-methyl Serine and alpha-methyl Tyrosine.
4. The synthetic peptide of any one of embodiments 1-3, wherein the synthetic peptide is substantially resistant to proteolytic degradation.
5. The synthetic peptide of embodiment 4, wherein the synthetic peptide is substantially resistant to DPP-IV, neprilysin, chymotrypsin, plasmin, thrombin, kallikrein, trypsin, elastase and/or pepsin degradation.
6. The synthetic peptide of any one of embodiments 1-5, wherein the native amino acid residueis a site susceptible to proteolytic cleavage.
7. The synthetic peptide of any one of embodiments 1-6, wherein the peptide is an incretin class peptide.
8. The synthetic peptide of embodiment 7, wherein the peptide is selected from the group consisting of a glucagon-like peptide 1 (GLP-1), a glucose-dependent insulinotropic peptide (GIP), an exenatide peptide plus glucagon, secretins, tenomodulin, oxyntomodulin and vasoactive intestinal peptide (VIP).
9. The synthetic peptide of embodiment 1, wherein the peptide is insulin.
10. A synthetic GLP-1 peptide comprising at least three substitutions of alpha-methyl functionalized amino acids for native amino acid residues, wherein the synthetic GLP-1 peptide maintains substantially the same receptor potency as a corresponding synthetic GLP-1 peptide that does not comprise the substitutions.
11. The synthetic GLP-1 peptide of embodiment 10, wherein the at least three alpha-methyl functionalized amino acids are alpha-methyl Phenylalanine.
12. The synthetic GLP-1 peptide of embodiment 10, comprising four alpha-methyl functionalized amino acids.
13. The synthetic GLP-1 peptide of embodiment 12, wherein the four alpha-methyl functionalized amino acids are alpha-methyl Phenylalanine substituted at positions Phe6, Try13, Phe22 and Trp25.
14. The synthetic GLP-1 peptide of any one of embodiments 10-13, further comprising an aminoisobutyric acid substitution at position 2 (Aib2).
15. The synthetic GLP-1 peptide of any one of embodiments 10-14, further comprising a serine modification at position 5 (Ser5).
16. The synthetic GLP-1 peptide of any one of embodiments 10-15, further comprising an alpha-methyl Lysine substituted at positions Lys20 and Lys28.
17. The synthetic GLP-1 peptide of any one of embodiments 10-16, further comprising a Valine substituted for Leucine26.
18. The synthetic GLP-1 peptide of any one of embodiments 10-17, further comprising a C-terminal lipidation.
19. The synthetic GLP-1 peptide of any one of embodiments 10-18 wherein the synthetic GLP-1 peptide is substantially resistant to proteolytic degradation.
20. The synthetic GLP-1 peptide of embodiment 19, wherein the synthetic GLP-1 peptide is substantially resistant to DPP-IV, neprilysin, chymotrypsin, plasmin, thrombin, kallikrein, trypsin, elastase and/or pepsin degradation.
21. A method of preparing a synthetic peptide, comprising:
   a. identifying at least one native amino acid residue in the peptide for substitution; and
   b. substituting an alpha-methyl functionalized amino acid for the identified native amino acid residue,
   wherein the synthetic peptide maintains substantially the same receptor potency and selectivity as a corresponding synthetic peptide that does not comprise the substitution, and
   wherein the synthetic peptide is substantially resistant to proteolytic degradation.
22. The method of embodiment 21, wherein the substituted alpha-methyl functionalized amino acid corresponds to the substituted native amino acid residue.
23. The method of embodiment 21, wherein the substituted alpha-methyl functionalized amino acid is alpha-methyl phenylalanine.
24. The method of any one of embodiments 21-23, wherein the synthetic peptide is substantially resistant to DPP-IV, neprilysin, chymotrypsin, plasmin, thrombin, kallikrein, trypsin, elastase and/or pepsin degradation.
25. The method of embodiment 21, wherein the identifying comprises identifying amino acids at sites susceptible to enzymatic cleavage.
26. The method of embodiment 21, wherein the peptide is an incretin class peptide.
27. The method of embodiment 26, wherein the peptide is selected from the group consisting of a glucagon-like peptide 1 (GLP-1), glucagon, a glucose-dependent insulinotropic peptide (GIP), and an exenatide peptide.
28. The method of embodiment 21, wherein the peptide is insulin.
29. A method of preparing a proteolytically stable peptide, comprising:
   a. exposing a peptide to one or more proteases;
   b. identifying at least one native amino acid residue which is a site susceptible to proteolytic cleavage; and
   c. substituting an alpha-methyl functionalized amino acid for the identified amino acid residue,
   wherein the synthetic peptide maintains substantially the same receptor potency and selectivity as a corresponding synthetic peptide that does not comprise the substitution, and
   wherein the synthetic peptide is substantially resistant to proteolytic degradation.
30. The method of embodiment 29, wherein the substituted alpha-methyl functionalized amino acid corresponds to the substituted native amino acid residue.
31. The method of embodiment 29, wherein the substituted alpha-methyl functionalized amino acid is alpha-methyl phenylalanine.
32. The method of any one of embodiments 29-31, wherein the synthetic peptide is substantially resistant to DPP-IV, neprilysin, chymotrypsin, plasmin, thrombin, kallikrein, trypsin, elastase and/or pepsin degradation.
33. The method of embodiment 32, wherein the peptide is an incretin class peptide.
34. The method of embodiment 33, wherein the peptide is selected from the group consisting of a glucagon-like peptide 1 (GLP-1), a glucose-dependent insulinotropic peptide (GIP), and an exenatide peptide.
35. The method of embodiment 29, wherein the peptide is insulin.
36. A method of treating a patient comprising administering a pharmaceutically effective amount of a synthetic peptide of embodiment 1 to the patient.
37. A method of treating a patient diagnosed with diabetes comprising administering a therapeutically effective amount of the synthetic GLP-1 peptide of embodiment 10 to the patient.
38. A method of treating a patient diagnosed with diabetes comprising administering a therapeutically effective amount of the synthetic insulin of embodiment 9 to the patient.
39. The methods of any one of embodiments 36-38, wherein the administration is oral.
40. A synthetic GLP-1 peptide comprising the following amino acid sequence:

   R¹-His-X1-Glu-Gly-X2-X3-Thr-Ser-Asp-Val-Ser-Ser-X4-Leu-Glu-Gly-Gln-Ala-Ala-X5-Glu-X6-Ile-Ala-X7-X8-X9-X10-X11-X12-R² (SEQ ID NO:2)

   , wherein:
   R¹ is Hy, Ac or *p*Glu;
   R² is -NH₂ or -OH;
   X1 is Ala, Aib, Pro or Gly;
   X2 is Thr, Pro or Ser;
   X3 is Aib, Bip, β,β-Dip, F5-Phe, Phe, PhG, Nle, homoPhe, homoTyr, *N*-MePhe, α-MePhe, α-Me-2F-Phe, Tyr, Trp, Tyr-OMe, 4I-Phe, 2F-Phe, 3F-Phe, 4F-Phe, 1-NaI, 2-NaI, Pro or di-β,β-Me-Phe;
   X4 is Aib, Ala, Asp, Arg, Bip, Cha, β,β-Dip, Gln, F5-Phe, PhG, Nle, homoPhe, homoTyr, α-MePhe, α-Me-2F-Phe, Phe, Thr, Trp, Tyr-OMe, 4I-Phe, 2F-Phe, 3F-Phe, 4F-Phe, Tyr, 1-NaI, 2-NaI, Pro or di-β,β-Me-Phe;
   X5 is Aib, Lys, D-pro, Pro or α-MeLys;
   X6 is Aib, Asp, Arg, Bip, Cha, Leu, Lys, 2Cl-Phe, 3Cl-Phe, 4Cl-Phe, PhG, homoPhe, 2Me-Phe, 3Me-Phe, 4Me-Phe, 2CF₃-Phe, 3CF₃-Phe, 4CF₃-Phe, β-Phe, β-MePhe, D-phe, 4I-Phe, 3I-Phe, 2F-Phe, β,β-Dip, β-Ala, Nle, Leu, F5-Phe, homoTyr, α-MePhe, α-Me-2F-Phe, Ser, Tyr, Trp, Tyr-OMe, 3F-Phe, 4F-Phe, Pro, 1-NaI, 2-NaI or di-β,β-Me-Phe;
   X7 is Aib, Arg, Bip, Cha, β,β-Dip, F5-Phe, PhG, Phe, Tyr, homoPhe, homoTyr, α-MePhe, α-Me-2F-Phe, 2Me-Phe, 3Me-Phe, 4Me-Phe, Nle, Tyr-OMe, 4I-Phe, 1-NaI, 2-NaI, 2F-Phe, 3F-Phe, 4F-Phe, Pro, *N*-MeTrp, α-MeTrp or di-β,β-Me-Phe;
   X8 is Aib, Ala, Arg, Asp, Glu, Nle, Pro, Ser, *N*-MeLeu, α-MeLeu or Val;
   X9 is Aib, Glu, Lys, α-MeVal or Pro;
   X10 is Aib, Glu, α-MeLys or Pro;
   X11 is Aib, Glu, Pro or Ser; and
   X12 is Aib, Gly, Glu, Pro or α-MeArg.

## Claims

1. A method of preparing a proteolytically stable GLP-1 peptide analog, comprising substituting an alpha-methyl functionalized phenylalanine in at least one of positions 6, 13, 22, or 25 for the GLP-1 amino acid residue,
wherein the synthetic GLP-1 peptide maintains substantially the same receptor potency and selectivity as a corresponding synthetic peptide that does not comprise the at least one substitution, and
wherein the synthetic peptide is substantially resistant to proteolytic degradation.

2. A synthetic GLP-1 peptide comprising the following amino acid sequence:
R¹-His-X1-Glu-Gly-X2- α-MePhe -Thr-Ser-Asp-Val-Ser-Ser- α-MePhe -Leu-Glu-Gly-Gln-Ala-Ala-X5-Glu- α-MePhe -Ile-Ala- α-MePhe -X8-X9-X10-X11-X12-R²,
wherein:
R¹ is Hy, Ac or *p*Glu;
R² is -NH₂ or -OH;
X1 is Ala, Aib, Pro or Gly;
X2 is Thr, Pro or Ser;
X5 is Aib, Lys, D-pro, Pro or α-MeLys;
X8 is Aib, Ala, Arg, Asp, Glu, Nle, Pro, Ser, *N*-MeLeu, α-MeLeu or Val;
X9 is Aib, Glu, Lys, α-MeVal or Pro;
X10 is Aib, Glu, α-MeLys or Pro;
X11 is Aib, Glu, Pro or Ser; and
X12 is Aib, Gly, Glu, Pro or α-MeArg.

3. The GLP-1 peptide of claim 2 for use in a method of treating a patient, wherein a pharmaceutically effective amount of the peptide is administered to the patient.

4. The GLP-1 peptide of claim 2 for use in a method of treating a patient diagnosed with diabetes, wherein a therapeutically effective amount of the peptide is administered to the patient.

5. The GLP-1 peptide for use of any one of claims 3 or 4, wherein the administration is oral.
